# EUROPEAN PATENT SPECIFICATION

(11) **EP 3 868 299 B1**
(45) Date of publication and mention of the grant of the patent: **22.11.2023**
(21) Application number: 19872446.0
(22) Date of filing: 30.09.2019
(51) Int. Cl.: A61B 6/00, A61B 6/02, A61B 6/04

(54) **RADIOLOGICAL INTERPRETATION SUPPORT APPARATUS, OPERATION PROGRAM THEREOF, AND OPERATION METHOD THEREOF**
VORRICHTUNG ZUR UNTERSTÜTZUNG DER RADIOLOGISCHEN INTERPRETATION, BETRIEBSPROGRAMM DAVON UND BETRIEBSVERFAHREN DAFÜR
APPAREIL DE SUPPORT D'INTERPRÉTATION RADIOLOGIQUE, PROGRAMME DE FONCTIONNEMENT CORRESPONDANT ET PROCÉDÉ DE FONCTIONNEMENT CORRESPONDANT

(30) Priority: 19.10.2018 JP 2018197541
(43) Date of publication of application: 25.08.2021
(73) Proprietor: FUJIFILM Corporation, Tokyo 106-8620 (JP)
(72) Inventor: FUKUDA, Wataru, Ashigarakami-gun, Kanagawa 258-0023 (JP)
(74) Representative: Kudlek, Franz Thomas
(86) International application number: PCT/JP2019/038572
(87) International publication number: WO 2020/080082

(56) References cited:
- WO-A1-2012/107057
- JP-A- 2017 047 079
- JP-A- 2017 047 079
- JP-A- 2017 047 080
- JP-A- 2018 043 001
- JP-A- 2018 043 001
- US-A1- 2011 123 087
- US-A1- 2015 269 766
- US-A1- 2016 110 875

## Description

### Technical Field

The technology of the present disclosure relates to an image interpretation support apparatus, a non-transitory storage medium storing an operation program, and an operation method.

### Related Art

In a mammography apparatus which images a breast as a subject, tomosynthesis imaging is performed. In the tomosynthesis imaging, a radiation source is moved to a plurality of positions with respect to a radiation detector, and radiation is emitted from the radiation source at each position. Then, a plurality of tomographic images in a plurality of tomographic planes of the breast are generated from a plurality of projection images obtained in this manner.

In the image interpretation, a user such as a radiologist does not immediately interpret a tomographic image, but first interprets a two-dimensional standard image. The user roughly guesses a location of a lesion part such as calcification in the two-dimensional standard image. Then, the user searches for a tomographic image of a tomographic plane where the guessed lesion part is likely to be present, and interprets the searched tomographic image in detail. Such an image interpretation procedure is used because it is inefficient to randomly interpret a plurality of tomographic images without any guess at first.

The two-dimensional standard image is, for example, a simple imaging image obtained by so-called simple imaging in which a radiation source faces a radiation detector (the radiation source is arranged on a normal line passing through the center of a detection surface of the radiation detector to face the detection surface) to emit the radiation. In the simple imaging, there are craniocaudal view (CC) imaging in which the breast is imaged by being vertically sandwiched and compressed, and mediolateral oblique view (MLO) imaging in which the breast is imaged by being obliquely sandwiched at an angle of about 60° and compressed. Hereinafter, the simple imaging image obtained by the CC imaging is referred to as a CC image, and the simple imaging image obtained by the MLO imaging is referred to as an MLO image.

WO2014/203531A discloses a technology to save labor of searching for tomographic images during image interpretation. That is, the two-dimensional standard image is divided into a plurality of regions, and similarly, each tomographic image is divided into a plurality of regions. Then, a correlation between the region of the two-dimensional standard image and the region of each tomographic image is obtained, and a tomographic plane of the tomographic image having the region with the largest correlation is specified as a corresponding tomographic plane which corresponds to the region of the two-dimensional standard image.

More specifically, a correlation between a region R_S of a two-dimensional standard image S and a region R_Ti of each tomographic image Ti (i = 1 to N, N is the number of tomographic images) having a positional relationship corresponding thereto. Then, in a case where the region with the largest correlation with the region R_S is, for example, a region R_T10 of a tomographic image T10, a tomographic plane TF10 of the tomographic image T10 is specified as a corresponding tomographic plane of the region R_S. The corresponding tomographic plane is specified for each region. Information on the corresponding tomographic plane specified in this manner and the region of the two-dimensional standard image are stored as correspondence information (indicated as a depth map in WO2014/203531A).

Next, on a display screen of the two-dimensional standard image, a selection instruction of a location on the two-dimensional standard image by the user is accepted. The corresponding tomographic plane of the region in which the location selected by the selection instruction is present is read out from the correspondence information, and the tomographic image of the read corresponding tomographic plane is displayed on the display screen together with the two-dimensional standard image.

In "Regarding image interpretation function for both X-rays and ultrasonic waves in mammography system" published in October 2016, JIRA Technical Report 2016. Vol.26 No.2 (Vol. 51) p38-p42, in order to be useful for ultrasonography after mammography, palpation, or the like, a technology of estimating a location selected by the selection instruction from the user on the CC image and the MLO image and displaying a mark indicating the estimated location on a breast schema diagram is described on the Internet <URL:http://www.jira-net.or.jp/publishing/files/74/jira_technical_report_51.pdf> (hereinafter, referred to as non-patent literature).

More specifically, first, the contour of the breast is extracted from the CC image and the MLO image to estimate a nipple position. Then, a distance between a line that passes through the estimated nipple position and is perpendicular to the side of the chest wall and the location selected by the selection instruction from the user is obtained. A mark is displayed at a position of the schema diagram away from the line by the obtained distance.

Document JP2017047079A may be considered to disclose an image interpretation support apparatus comprising: a reception unit that receives a selection instruction of a location on one two-dimensional image having information on a breast; an acquisition unit that acquires information on a tomographic plane which is the location of the selection instruction received by the reception unit, among a plurality of tomographic planes of the breast, the tomographic plane being specified on the basis of a plurality of tomographic images in the plurality of tomographic planes obtained by tomosynthesis imaging of the breast; and a generation unit that generates a schema diagram of the breast with a mark indicating the selected location on the basis of the information on the tomographic plane acquired by the acquisition unit. The document may also be considered to disclose the corresponding storage medium storing an operation program, and the corresponding operation method.

### SUMMARY OF INVENTION

### Technical Problem

In the non-patent literature, as described above, the user has to make a selection instruction for the location on both the CC image and the MLO image. Therefore, it is necessary for the user to make a selection instruction by finding the same location as the location that has been selected by the selection instruction on one image of the CC image and the MLO image, from the other image. In this manner, in the non-patent literature, the burden is put on the user.

### Solution to Problem

An object of the technology of the present disclosure is to provide an image interpretation support apparatus, a non-transitory storage medium storing an operation program, and an operation method, which can reduce the burden on the user.

In order to achieve the object, an image interpretation support apparatus of the present disclosure includes a reception unit that receives a selection instruction of a location on one two-dimensional standard image having information on a breast; an acquisition unit that acquires information on a corresponding tomographic plane corresponding to a selected location, which is the location of the selection instruction received by the reception unit, among a plurality of tomographic planes of the breast, the corresponding tomographic plane being specified on the basis of a plurality of tomographic images in the plurality of tomographic planes obtained by tomosynthesis imaging of the breast; and a generation unit that generates a schema diagram of the breast with a mark indicating the selected location on the basis of the information on the corresponding tomographic plane acquired by the acquisition unit.

It is preferable that the image interpretation support apparatus includes a specifying unit that specifies the corresponding tomographic plane on the basis of the plurality of tomographic images.

It is preferable that the specifying unit specifies the corresponding tomographic plane for each location of the two-dimensional standard image before receiving the selection instruction at the reception unit, and the image interpretation support apparatus further includes a storage controller that performs control to store, as correspondence information, the information on the corresponding tomographic plane specified in the specifying unit and a location of the corresponding tomographic plane specified by the specifying unit, in a storage unit, and that outputs the information on the corresponding tomographic plane corresponding to the selected location to the acquisition unit by reading the information on the corresponding tomographic plane from the correspondence information.

It is preferable that the specifying unit specifies the corresponding tomographic plane corresponding to the selected location in a case in which the reception unit receives the selection instruction.

It is preferable that the generation unit determines a display position of the mark on the basis of a distance between a line, which passes through a nipple position shown in the two-dimensional standard image and is perpendicular to a side of a chest wall, and the selected location.

It is preferable that the image interpretation support apparatus includes an estimation unit that estimates a compression rate of the breast, and the generation unit determines a display position of the mark on the basis of an estimation result of the estimation unit.

It is preferable that the two-dimensional standard image is at least one of a craniocaudal view image obtained by imaging the breast in a craniocaudal direction, a mediolateral oblique view image obtained by imaging the breast in a mediolateral oblique direction, a composite craniocaudal view image generated on the basis of a plurality of craniocaudal view tomographic images obtained by the tomosynthesis imaging of the breast in a craniocaudal view imaging method, or a composite mediolateral oblique view image generated on the basis of a plurality of mediolateral oblique view tomographic images obtained by the tomosynthesis imaging of the breast in a mediolateral oblique view imaging method.

It is preferable that the generation unit determines a display position of the mark on the basis of information on an imaging angle in the mediolateral oblique view imaging in a case in which the two-dimensional standard image is the mediolateral oblique view image or the composite mediolateral oblique view image.

It is preferable that the generation unit changes the schema diagram in accordance with a size and a shape of the breast.

It is preferable that the image interpretation support apparatus further includes a detection unit that detects a type of a structure shown in the selected location, and the generation unit changes the mark in accordance with the type detected by the detection unit.

It is preferable that the image interpretation support apparatus further includes a display controller that performs control to display the schema diagram on a display unit.

It is preferable that the display controller performs control to display, in addition to the schema diagram, on the display unit, the tomographic image of the corresponding tomographic plane among the tomographic images obtained by the tomosynthesis imaging of the breast in the same imaging method as an imaging method of the two-dimensional standard image.

It is preferable that the display controller performs control to further display, on the display unit, an image obtained by imaging the breast in a different imaging method from the imaging method of the two-dimensional standard image. It is preferable that the display controller displays an estimated region that is estimated to include the selected location, on the image obtained by imaging the breast in the different imaging method from the imaging method of the two-dimensional standard image.

In order to achieve the object, a non-transitory storage medium storing an operation program that causes a computer to perform operation processing of an image interpretation support apparatus is provided, the operation processing includes: receiving a selection instruction of a location on one two-dimensional standard image having information on a breast; acquiring information on a corresponding tomographic plane corresponding to a selected location, which is the location of the received selection instruction, among a plurality of tomographic planes of the breast, the corresponding tomographic plane being specified on the basis of a plurality of tomographic images in the plurality of tomographic planes obtained by tomosynthesis imaging of the breast; and generating a schema diagram of the breast with a mark indicating the selected location on the basis of the information on the acquired corresponding tomographic plane.

An operation method of an image interpretation support apparatus of the present disclosure includes receiving a selection instruction of a location on one two-dimensional standard image having information on a breast; acquiring information on a corresponding tomographic plane corresponding to a selected location, which is the location of the received selection instruction, among a plurality of tomographic planes of the breast, the corresponding tomographic plane being specified on the basis of a plurality of tomographic images in the plurality of tomographic planes obtained by tomosynthesis imaging of the breast; and generating a schema diagram of the breast with a mark indicating the selected location on the basis of the information on the acquired corresponding tomographic plane.

### Effects of Invention

According to the technology of the present disclosure, it is possible to provide an image interpretation support apparatus and an operation program and an operation method thereof which can reduce the burden on the user.

### BRIEF DESCRIPTION OF THE DRAWINGS

Fig. 1 is a diagram illustrating a medical system.
Fig. 2 is a diagram illustrating a state of CC imaging.
Fig. 3 is a diagram illustrating a state of MLO imaging.
Fig. 4 is a diagram illustrating a state of tomosynthesis imaging.
Fig. 5 is a diagram illustrating a state in which a plurality of tomographic images are generated from a plurality of projection images obtained in the tomosynthesis imaging.
Fig. 6 is a flowchart illustrating a procedure of imaging a breast by a mammography apparatus.
Fig. 7 is a flowchart illustrating a procedure of imaging the breast by the mammography apparatus.
Fig. 8 is a diagram illustrating an image set.
Fig. 9 is a block diagram illustrating a computer constituting an image interpretation support apparatus.
Fig. 10 is a block diagram illustrating a processing unit of a CPU of the image interpretation support apparatus.
Fig. 11 is a diagram illustrating a location of a CC image for specifying a CC corresponding tomographic plane by a specifying unit.
Fig. 12 is a diagram illustrating a location of an MLO image for specifying an MLO corresponding tomographic plane by the specifying unit.
Fig. 13 is a diagram illustrating a state in which the corresponding tomographic plane is specified by the specifying unit.
Fig. 14 is a diagram illustrating correspondence information.
Fig. 15 is a diagram illustrating a schema diagram template.
Fig. 16 is a flowchart illustrating a procedure of generating a schema diagram with a mark indicating a selected location in a generation unit.
Figs. 17A and 17B are diagrams illustrating a state of calculating a distance between a line that passes through a nipple position and is perpendicular to a side of a chest wall, and a selected location, Fig. 17A illustrates a case of the CC image, and Fig. 17B illustrates a case of the MLO image.
Fig. 18 is a diagram for describing a lateral compression rate and a vertical compression rate.
Figs. 19A and 19B are diagrams illustrating a state of displaying a mark on the schema diagram, Fig. 19A illustrates a case of the CC image, and Fig. 19B illustrates a case of the MLO image.
Fig. 20 is a diagram illustrating a transmission instruction acceptance screen.
Fig. 21 is a diagram illustrating an image display screen.
Fig. 22 is a diagram illustrating a selection instruction acceptance screen.
Fig. 23 is a diagram illustrating a schema diagram display screen.
Fig. 24 is a diagram illustrating a schema diagram display screen on which a line indicating a correspondence relationship between a mark and a cursor is displayed.
Fig. 25 is a flowchart illustrating a processing procedure of the image interpretation support apparatus.
Fig. 26 is a flowchart illustrating a processing procedure of the image interpretation support apparatus.
Fig. 27 is a block diagram illustrating a processing unit of a CPU of an image interpretation support apparatus of a second embodiment.
Fig. 28 is a flowchart illustrating a processing procedure of the image interpretation support apparatus of the second embodiment.
Fig. 29 is a diagram illustrating a third embodiment in which a compression rate of the breast is estimated and a display position of a mark is decided on the basis of the estimation result.
Fig. 30 is a diagram illustrating a fourth embodiment in which a display position of a mark is decided on the basis of information on an imaging angle in MLO imaging.
Fig. 31 is a diagram illustrating a schema diagram template of a fifth embodiment.
Fig. 32 is a diagram illustrating the fifth embodiment in which a schema diagram is changed in accordance with the size and shape of the breast.
Fig. 33 is a diagram illustrating a sixth embodiment in which a type of a structure shown in a selected location is detected and a mark is changed in accordance with the detected type.
Fig. 34 is a diagram illustrating a schema diagram display screen of a seventh embodiment.
Fig. 35 is a diagram illustrating an eighth embodiment in which a composite CC image and a composite MLO image are generated to be used as a two-dimensional standard image.
Fig. 36 is a diagram conceptually illustrating a minimum intensity projection method.

### DESCRIPTION OF EMBODIMENTS

### [First Embodiment]

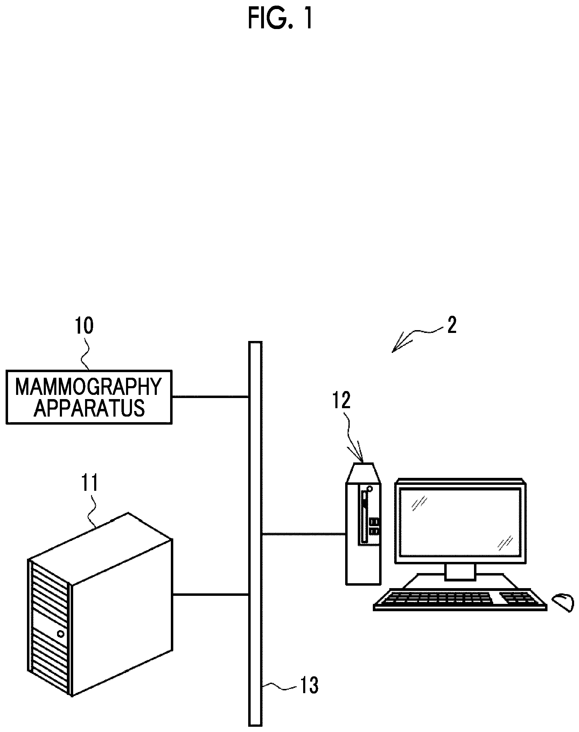

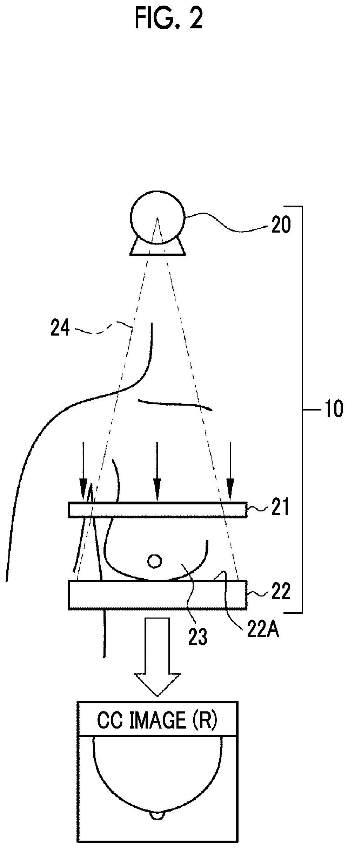

In Fig. 1, a medical system 2 comprises a mammography apparatus 10, a picture archiving and communication system (PACS) server 11, and an image interpretation support apparatus 12, and is installed at one medical facility, for example. As is well known, the mammography apparatus 10 emits radiation 24 (refer to Fig. 2 or the like) to a breast 23 (refer to Fig. 2 or the like), and outputs a radiographic image of the breast 23. The radiation 24 is, for example, X-rays (γ-rays are also possible). As is also well known, the PACS server 11 stores the radiographic image output from the mammography apparatus 10, and transmits the radiographic image to the image interpretation support apparatus 12. The image interpretation support apparatus 12 is operated by the user such as a radiologist. The mammography apparatus 10, the PACS server 11, and the image interpretation support apparatus 12 are connected so as to communicate with each other via a network 13 such as a local area network (LAN).

The PACS server 11 and the image interpretation support apparatus 12 are based on a computer such as a server computer, a workstation, or a personal computer. The PACS server 11 and the image interpretation support apparatus 12 are configured by installing a control program such as an operating system and various application programs on such a computer.

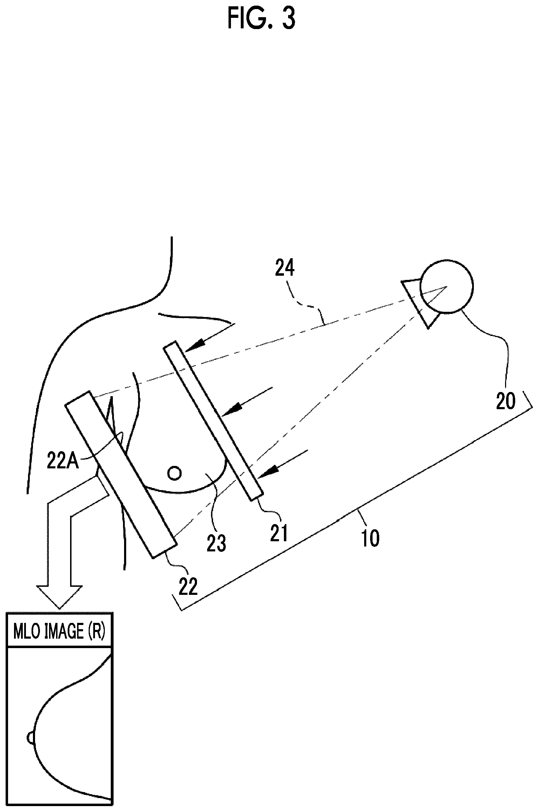

In Figs. 2 and 3, the mammography apparatus 10 has a radiation source 20, a compression plate 21, and a radiation detector 22. The radiation source 20 emits the radiation 24 to the breast 23 as indicated by two-dot chain lines. The compression plate 21 is formed of a material that transmits the radiation 24. The compression plate 21 pushes down the breast 23 in a vertical direction illustrated in Fig. 2 or in an obliquely downward direction illustrated in Fig. 3. The radiation detector 22 faces the compression plate 21, and compresses the breast 23 by vertically sandwiching the breast 23 with the compression plate 21 (Fig. 2) or compresses the breast 23 by obliquely sandwiching the breast 23 at an angle of about 60° with the compression plate 21 (Fig. 3). The radiation detector 22 detects the radiation 24 that is emitted from the radiation source 20 and is transmitted through the compression plate 21 and the breast 23, and outputs a radiographic image. The radiographic image output from the radiation detector 22 is transmitted to the PACS server 11 and is stored in the PACS server 11.

Fig. 2 illustrates a state of CC imaging in which the breast 23 is imaged by being vertically sandwiched and compressed, and which is simple imaging in which the radiation source 20 faces the radiation detector 22 (the radiation source 20 is arranged on a normal line passing through the center of a detection surface 22A of the radiation detector 22 to face the detection surface 22A) to emit the radiation 24. In this case, the radiation detector 22 outputs a CC image as the radiographic image. On the other hand, Fig. 3 illustrates a state of MLO imaging in which the breast 23 is imaged by being obliquely sandwiched and compressed, and which is also simple imaging. In this case, the radiation detector 22 outputs an MLO image as the radiographic image. The mammography apparatus 10 has an angle measurement function of measuring an angle at which the breast 23 is sandwiched (hereinafter, referred to as the imaging angle) in the MLO imaging. Although Figs. 2 and 3 illustrate the CC imaging and the MLO imaging for the right breast 23, the CC imaging and the MLO imaging are similarly performed for the left breast 23.

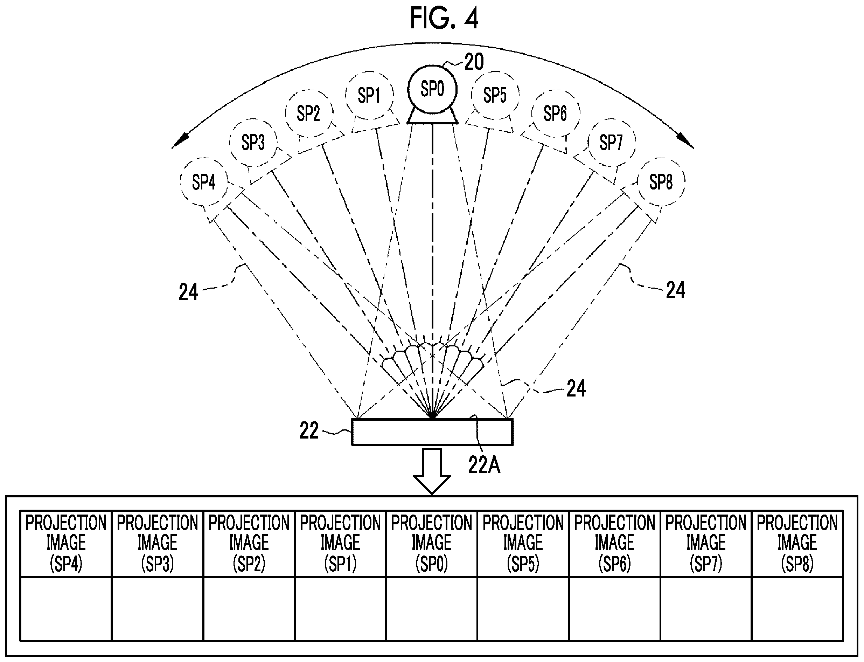

In Fig. 4 which conceptually illustrates a state of tomosynthesis imaging, the radiation source 20 is sequentially moved to a total of nine positions SP0 to SP8 arranged at equal angles in an arc shape with respect to the radiation detector 22. Then, the radiation 24 (not illustrated except for the positions SP0, SP4, and SP8) is emitted to the breast 23 at each of the positions SP0 to SP8. The radiation detector 22 detects the radiation 24 emitted at each of the positions SP0 to SP8, and outputs a projection image at each of the positions SP0 to SP8 as the radiographic image. The tomosynthesis imaging is performed in both the CC imaging method and the MLO imaging method, and is performed on each of the left and right breasts 23 in each of both the imaging methods. Here, the position SP0 is a position of the simple imaging, at which the radiation source 20 faces the radiation detector 22. The positions of the tomosynthesis imaging are not limited to the above-described nine positions. Further, the movement trajectory of the radiation source 20 is not limited to the above-described arc shape, and may be a linear shape parallel to the detection surface 22A of the radiation detector 22 or an elliptical arc shape.

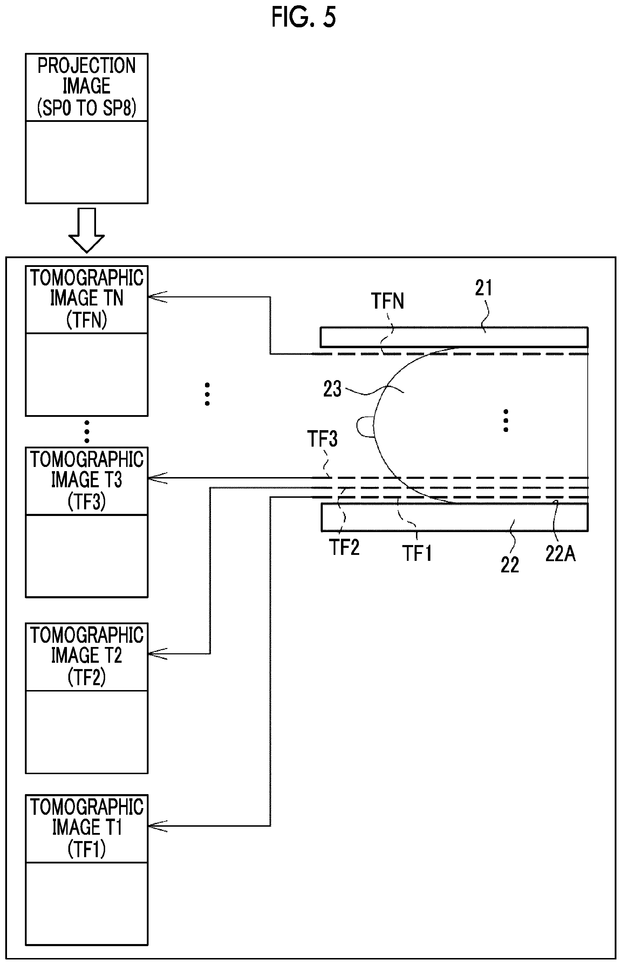

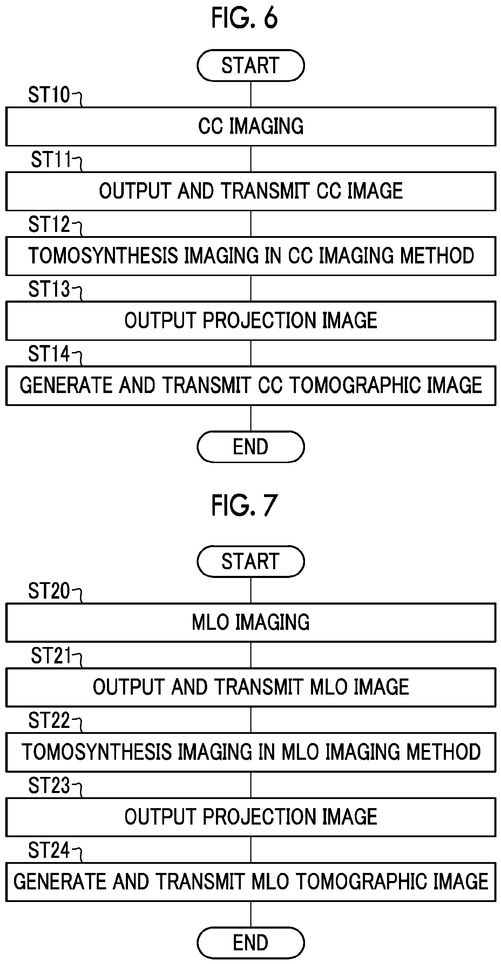

As illustrated in Fig. 5, the mammography apparatus 10 generates a plurality of tomographic images Ti (i = 1 to N, N is the number of tomographic images) from a plurality of projection images obtained by the tomosynthesis imaging illustrated in Fig. 4, by using a well-known method such as a filtered back projection method. The tomographic image Ti is an image in which a structure present in each of a plurality of tomographic planes TFi of the breast 23 is emphasized. The tomographic images Ti and the tomographic planes TFi have a one-to-one correspondence. Therefore, the same subscript i is used for the tomographic image Ti and the tomographic plane TFi. Each tomographic image Ti is transmitted to the PACS server 11, and is stored in the PACS server 11 in association with the CC image and the MLO image. In the following, the tomographic image obtained by the tomosynthesis imaging in the CC imaging method is expressed as a CC tomographic image (refer to Fig. 6 or the like), and the tomographic image obtained by the tomosynthesis imaging in the MLO imaging method is expressed as an MLO tomographic image (refer to Fig. 7 or the like).

The tomographic plane TFi is a plane parallel to the detection surface 22A of the radiation detector 22. An interval between adjacent tomographic planes TFi is, for example, 1 mm. The tomographic plane TF1 which is closest to the detection surface 22A of the radiation detector 22 is at a height of 10 mm from the detection surface 22A, for example. The tomographic plane TFN which is farthest from the detection surface 22A (closest to the radiation source 20) is at a height of 60 mm from the detection surface 22A (at a height of 50 mm from the tomographic plane TF1), for example. In this case, N = 51. The numerical values relating to the tomographic planes TFi described here are merely examples, and are not limited thereto.

Figs. 6 and 7 are flowcharts illustrating a procedure of imaging the breast 23 by the mammography apparatus 10. First, as illustrated in Fig. 6, in the mammography apparatus 10, the CC imaging illustrated in Fig. 2 is performed (Step ST10). In this manner, the CC image is output from the radiation detector 22, and is transmitted to the PACS server 11 (Step ST11).

Subsequently, in the mammography apparatus 10, the tomosynthesis imaging illustrated in Fig. 4 is performed in the CC imaging method (Step ST12). In this manner, a plurality of projection images are output from the radiation detector 22 (Step ST13). Then, as illustrated in Fig. 5, a plurality of CC tomographic images are generated from the plurality of projection images, and are transmitted to the PACS server 11 (Step ST14).

Similarly, as illustrated in Fig. 7, in the mammography apparatus 10, the MLO imaging illustrated in Fig. 3 is performed (Step ST20), and the MLO image is output from the radiation detector 22, and is transmitted to the PACS server 11 (Step ST21). Subsequently, the tomosynthesis imaging is performed in the MLO imaging method (Step ST22), the projection image is output (Step ST23), and the MLO tomographic image is generated and is transmitted to the PACS server 11 (Step ST24). These series of imaging procedures are performed for each of the left and right breasts 23. The order of CC imaging and MLO imaging may be reversed.

In the CC imaging and the MLO imaging, a relatively high amount of radiation 24 is emitted. On the other hand, in the tomosynthesis imaging in the CC imaging method and the tomosynthesis imaging in the MLO imaging method, a lower amount of radiation 24 than that in the CC imaging and the MLO imaging is emitted. Further, in the tomosynthesis imaging, the same dose of radiation 24 is emitted at each of the positions SP0 to SP8.

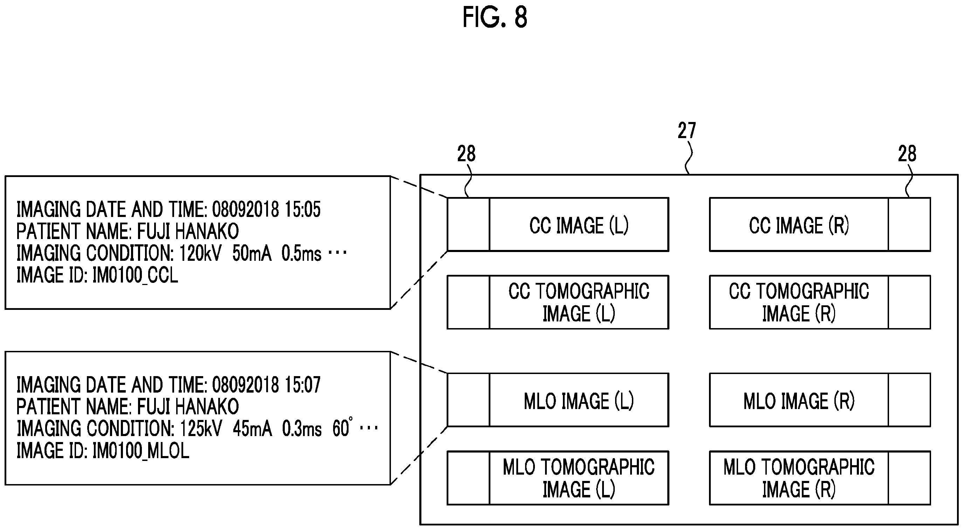

By performing imaging in the imaging procedures illustrated in Figs. 6 and 7, an image set 27 illustrated in Fig. 8 is stored in the PACS server 11. The image set 27 has a CC image (R) and a CC tomographic image (R) obtained by imaging the right breast 23 in the CC imaging method, and an MLO image (R) and an MLO tomographic image (R) obtained by imaging the right breast 23 in the MLO imaging method. Further, the image set 27 has a CC image (L) and a CC tomographic image (L) obtained by imaging the left breast 23 in the CC imaging method, and an MLO image (L) and an MLO tomographic image (L) obtained by imaging the left breast 23 in the MLO imaging method.

Each image constituting the image set 27 has accessory information 28 such as imaging date and time, a patient name, and an imaging condition. The imaging condition includes a tube voltage (120 kV or the like) applied to the radiation tube of the radiation source 20, a tube current (50 mA or the like), and an irradiation time (0.5 ms or the like) of the radiation 24. Further, in a case of the MLO image, the imaging angle (60° or the like) measured by the angle measurement function is also included. The images constituting the image set 27 have a common image identification data (ID) such as IM0100, and are associated with each other. Then, a symbol representing the type of each image, such as CCL of the CC image (L), is added to the common image ID so that the images are distinguished from each other. The image set 27 is transmitted to the image interpretation support apparatus 12. In the following, in a case where it is not necessary to distinguish the images from each other, (R) indicating the image of the right breast 23 and (L) indicating the image of the left breast 23 are not written. In addition to these images, the projection image which is the generation source of the tomographic image may be included in the image set 27. Further, instead of the tube current and the irradiation time, the tube current irradiation time product may be stored as the imaging condition.

In the embodiment, at least one image of the CC image or the MLO image is the two-dimensional standard image which has information on the breast 23 and on which a selection instruction of a location is accepted.

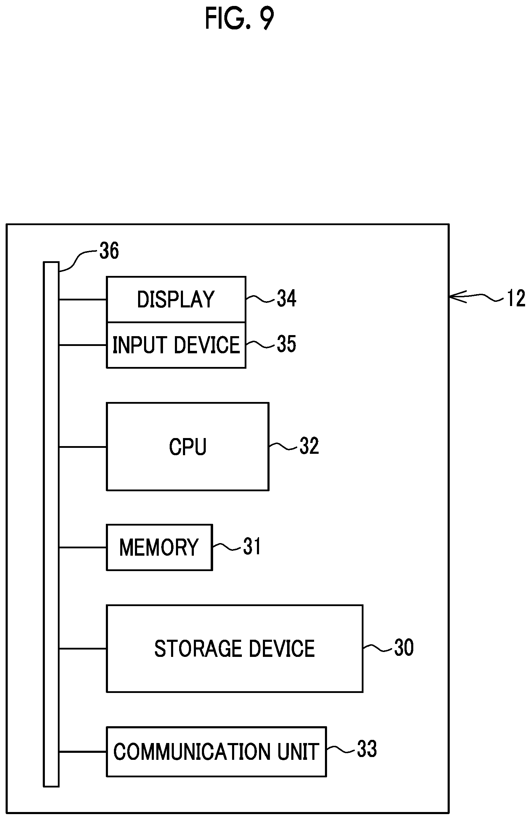

In Fig. 9, the computer constituting the image interpretation support apparatus 12 comprises a storage device 30, a memory 31, a central processing unit (CPU) 32, a communication unit 33, a display 34, and an input device 35. These are connected to each other through a data bus 36.

The storage device 30 is a hard disk drive that is built in the computer constituting the image interpretation support apparatus 12 or is connected to the computer constituting the image interpretation support apparatus 12 through a cable or a network. Alternatively, the storage device 30 is a disk array in which a plurality of hard disk drives are connected. Control programs such as an operating system, various application programs, and various kinds of data associated with these programs are stored in the storage device 30.

The memory 31 is a work memory required for the CPU 32 to execute processing. The CPU 32 performs overall control of each unit of the computer by loading a program stored in the storage device 30 to the memory 31 and executing the processing according to the program.

The communication unit 33 is a network interface to perform transmission control of various kinds of information through the network 13. The display 34 displays various screens. The various screens have an operation function based on the graphical user interface (GUI). The computer constituting the image interpretation support apparatus 12 accepts an input of an operation instruction from the input device 35 through the various screens. The input device 35 is a keyboard, a mouse, a touch panel, or the like.

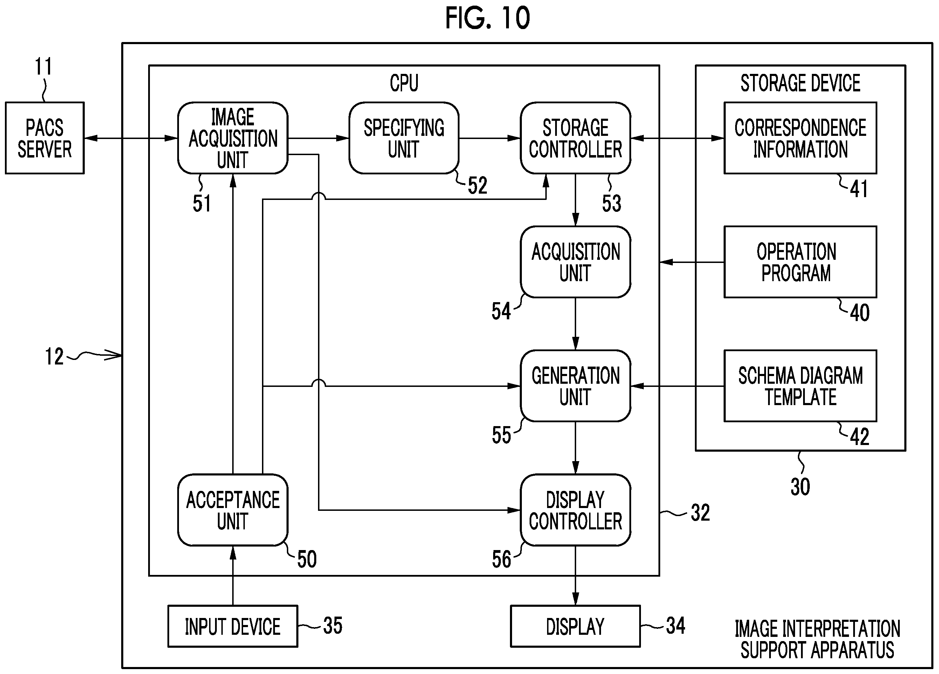

In Fig. 10, an operation program 40 is stored as the application program in the storage device 30 of the image interpretation support apparatus 12. The operation program 40 is an application program for causing the computer to function as the image interpretation support apparatus 12. That is, the operation program 40 is an example of an "operation program of the image interpretation support apparatus" according to the technology of the present disclosure. In addition to the operation program 40, correspondence information 41 and a schema diagram template 42 are stored in the storage device 30.

In a case where the operation program 40 is activated, the CPU 32 of the computer constituting the image interpretation support apparatus 12 cooperates with the memory 31 or the like to function as an acceptance unit 50, an image acquisition unit 51, a specifying unit 52, a storage controller 53, an acquisition unit 54, a generation unit 55, and a display controller 56.

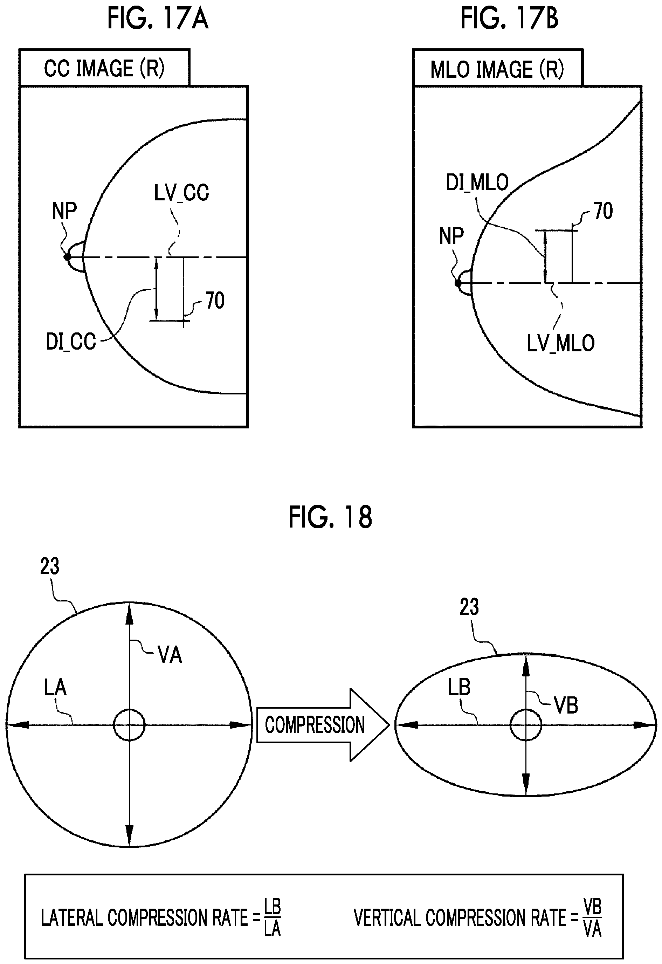

The acceptance unit 50 accepts an operation instruction input from the input device 35 through the various screens displayed on the display 34. As the operation instruction, there are a transmission instruction of the image set 27 to the PACS server 11, a selection instruction of a location on at least one image of the CC image or the MLO image, and the like. In the transmission instruction, information (image ID, patient name, imaging date and time, and the like) for uniquely identifying the image set 27 is included. In the selection instruction, positional information on a location 70 which is selected by the selection instruction (hereinafter, referred to as a selected location) (refer to Figs. 17A and 17B or the like) is included. The positional information is, for example, XY coordinates in a case where a pixel on the left end of the image is set as the origin, the lateral side of the image is set as an X axis, and the vertical side is set as a Y axis, and is XY coordinates of a pixel corresponding to the selected location 70. The acceptance unit 50 outputs the transmission instruction to the image acquisition unit 51, and outputs the selection instruction to the storage controller 53 and the generation unit 55.

The image acquisition unit 51 issues a transmission request for the image set 27 according to the transmission instruction from the acceptance unit 50, to the PACS server 11. In the transmission request, as in the transmission instruction, information for uniquely identifying the image set 27 is included. The image acquisition unit 51 acquires the image set 27 transmitted from the PACS server 11 in response to the transmission request. The image acquisition unit 51 outputs the acquired image set 27 to the specifying unit 52 and the display controller 56.

The specifying unit 52 specifies a CC corresponding tomographic plane on the basis of the plurality of CC tomographic images of the image set 27 from the image acquisition unit 51. Further, the specifying unit 52 specifies an MLO corresponding tomographic plane on the basis of the plurality of MLO tomographic images of the image set 27 from the image acquisition unit 51. Here, the CC corresponding tomographic plane is a tomographic plane corresponding to each location of the CC image. More specifically, the CC corresponding tomographic plane is a tomographic plane where a structure shown in each location of the CC image is present. Similarly, the MLO corresponding tomographic plane is a tomographic plane corresponding to each location of the MLO image, and is a tomographic plane where a structure shown in each location of the MLO image is present. The specifying unit 52 specifies one tomographic plane among the plurality of tomographic planes TFi of the breast 23 illustrated in Fig. 5, as the corresponding tomographic plane, for each location of the CC image. Similarly, the specifying unit 52 specifies one corresponding tomographic plane for each location of the MLO image. The specifying unit 52 outputs information on the specified CC corresponding tomographic plane and information on the specified MLO corresponding tomographic plane to the storage controller 53. The information on the corresponding tomographic plane is information indicating which of the plurality of tomographic planes TFi is the corresponding tomographic plane, and is, for example, the numerical value of i and a height from the detection surface 22A. Instead of the height from the detection surface 22A, a height from the tomographic plane TF1 may be used.

The storage controller 53 performs control to store, as the correspondence information 41, the information on the corresponding tomographic plane from the specifying unit 52 and the location corresponding thereto, in the storage device 30 as the storage unit. Further, the storage controller 53 reads the information on the corresponding tomographic plane corresponding to the selected location 70 selected by the selection instruction from the acceptance unit 50, from the correspondence information 41. The storage controller 53 outputs the read information on the corresponding tomographic plane to the acquisition unit 54.

The acquisition unit 54 acquires the information on the corresponding tomographic plane corresponding to the selected location 70, from the storage controller 53. The acquisition unit 54 outputs the acquired information on the corresponding tomographic plane to the generation unit 55.

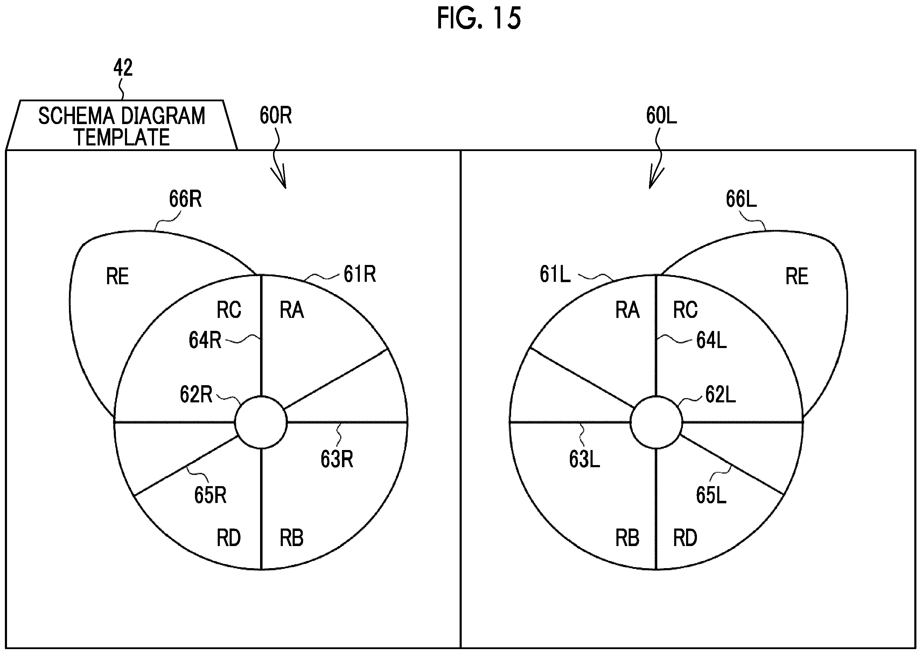

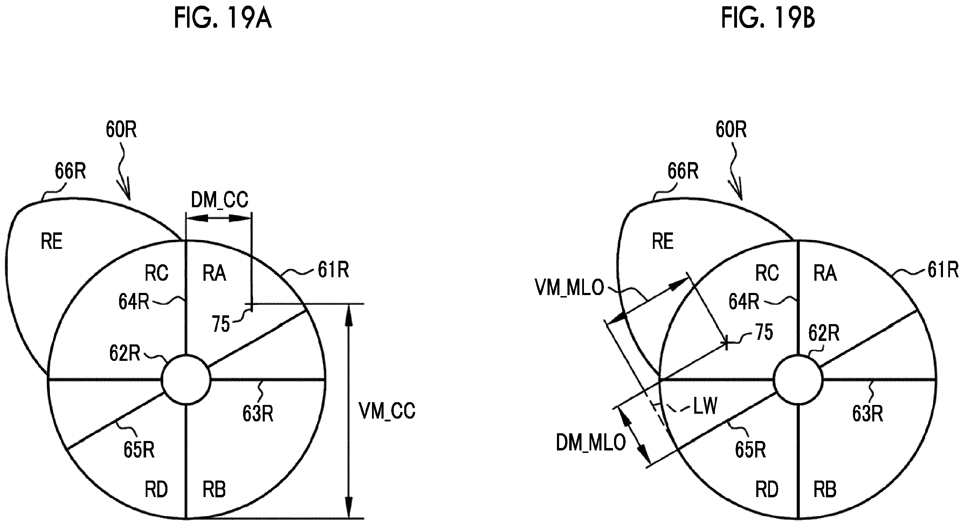

The generation unit 55 generates a schema diagram 60 (refer to Fig. 15 or the like) of the breast 23 with a mark 75 (refer to Figs. 19A and 19B or the like) indicating the selected location 70, on the basis of the positional information of the selected location 70 included in the selection instruction from the acceptance unit 50, the information on the corresponding tomographic plane from the acquisition unit 54, and the schema diagram template 42 of the storage device 30. The generation unit 55 outputs the generated schema diagram 60 to the display controller 56.

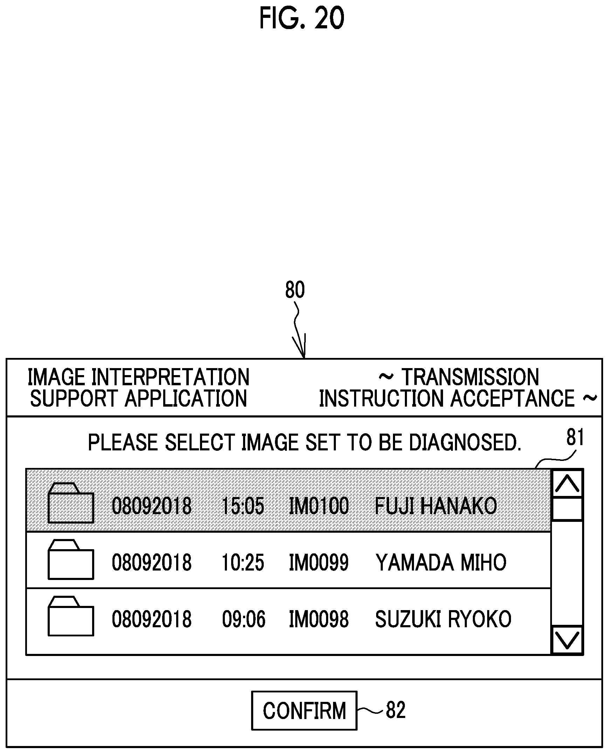

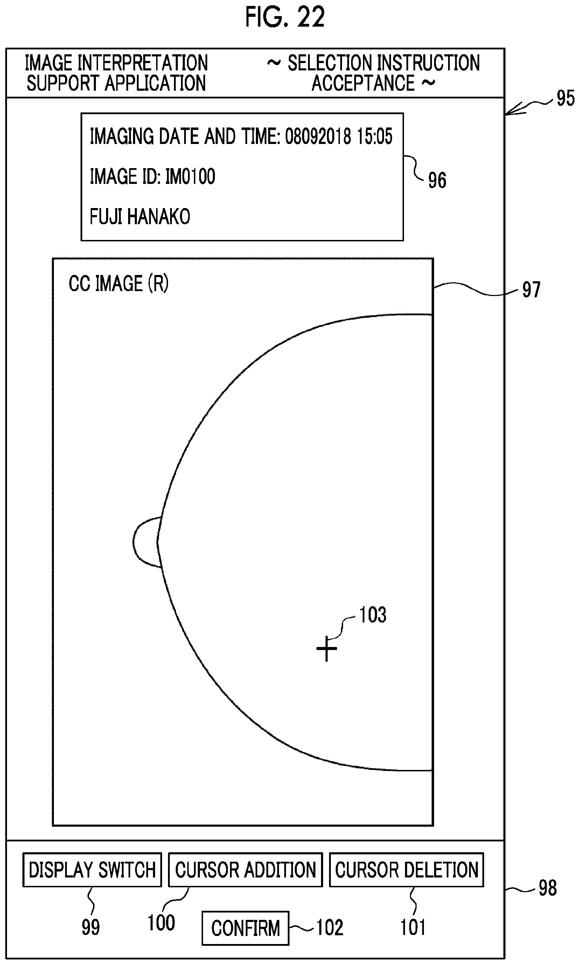

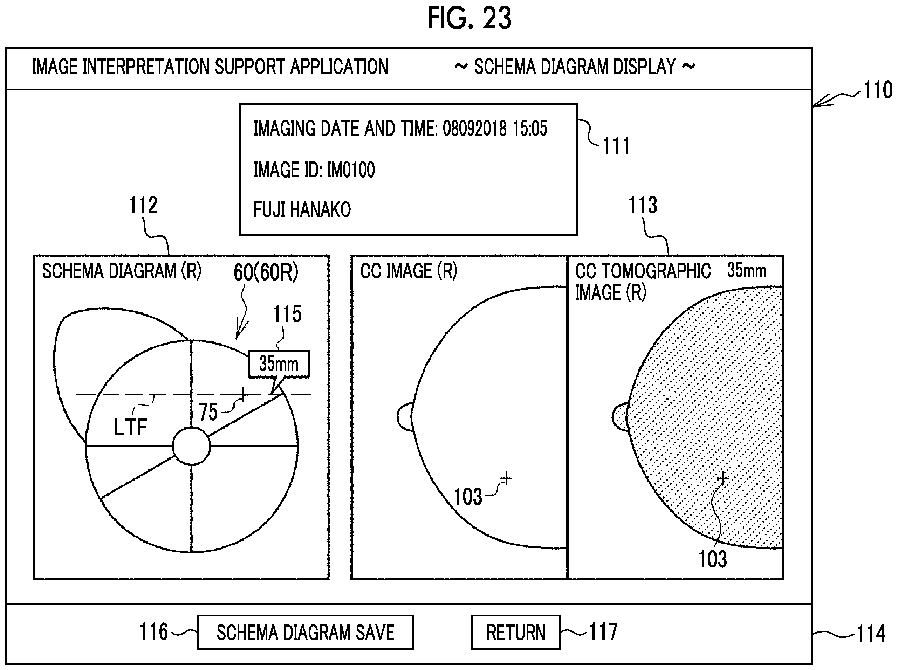

The display controller 56 performs control to display various screens on the display 34 as the display unit. Specifically, the display controller 56 performs control to display, on the display 34, a transmission instruction acceptance screen 80 (refer to Fig. 20) that accepts a transmission instruction, a selection instruction acceptance screen 95 (refer to Fig. 22) that accepts a selection instruction on at least one image of the CC image or the MLO image, a schema diagram display screen 110 (refer to Fig. 23) on which the schema diagram 60 with the mark 75 from the generation unit 55 is displayed, and the like.

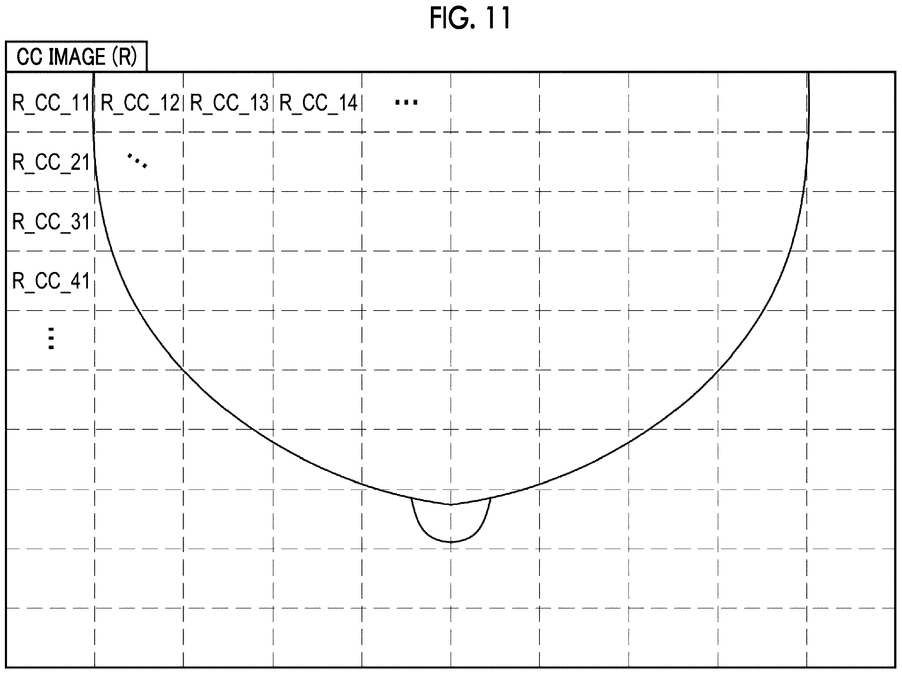

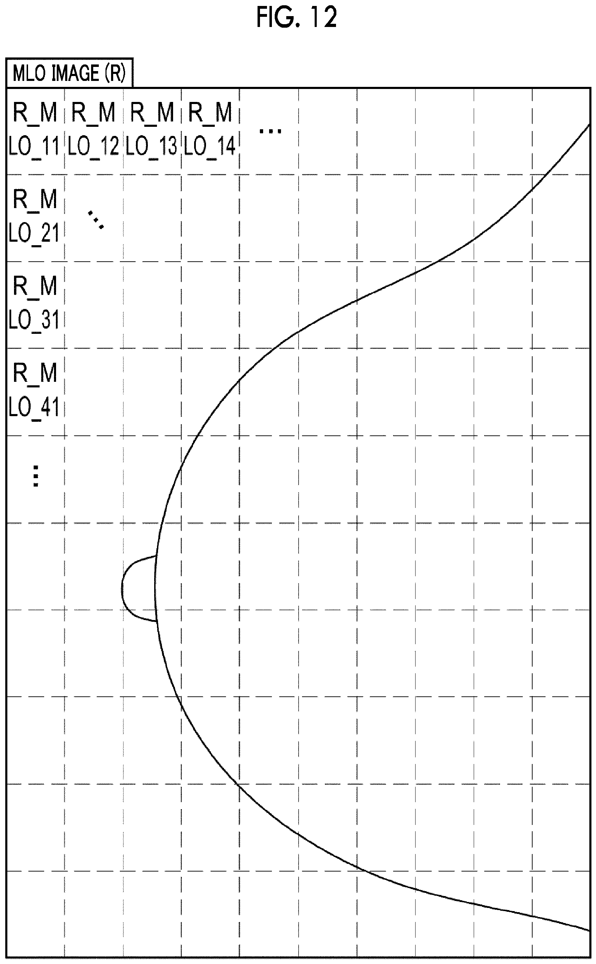

The location of the CC image which specifies the CC corresponding tomographic plane in the specifying unit 52 is a rectangular region R_CC_jk (j, k = 1 to 10) which is obtained by dividing the CC image into 10 equal parts respectively in the vertical and lateral directions and is composed of a plurality of pixels, as illustrated in Fig. 11. Similarly, the location of the MLO image which specifies the MLO corresponding tomographic plane in the specifying unit 52 is a rectangular region R_MLO_jk which is obtained by dividing the MLO image into 10 equal parts respectively in the vertical and lateral directions and is composed of a plurality of pixels, as illustrated in Fig. 12.

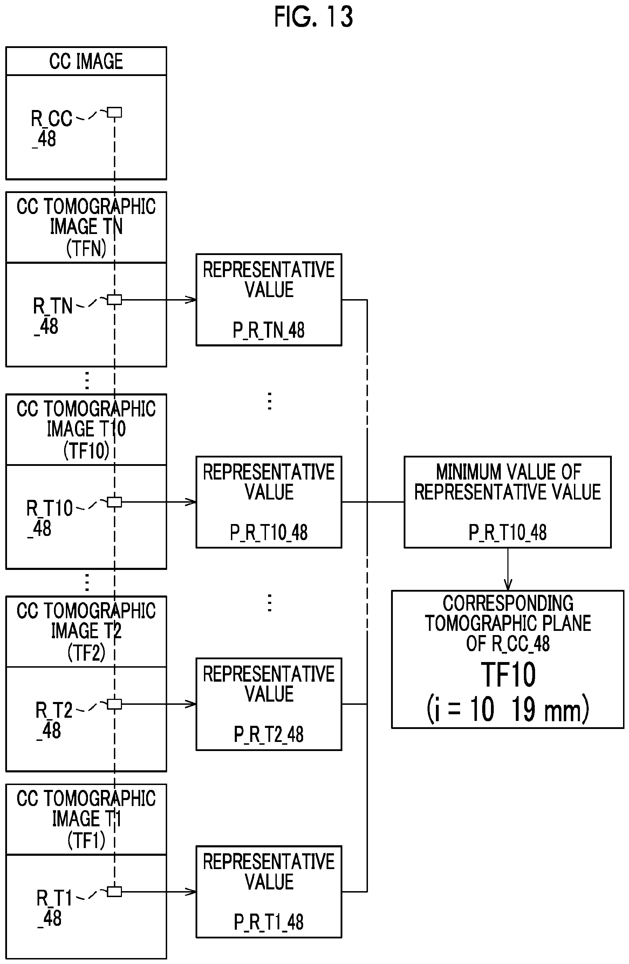

As illustrated in Fig. 13, the specifying unit 52 obtains a representative value P_R_Ti_jk of the pixel values, for the region R_Ti_jk of each CC tomographic image Ti having a positional relationship corresponding to the region R_CC_jk of the CC image. The representative value P_R_TiJk is an average value, mode, a minimum value, or the like of the pixel values of the region R_Ti_jk, for example.

The specifying unit 52 compares the magnitude of the representative values P_R_Ti_jk, and extracts a minimum value among the representative values P_R_Ti_jk. Then, the tomographic plane TFi of the CC tomographic image Ti having the region R_Ti_jk with the representative value P_R_Ti_jk that is the extracted minimum value, as the corresponding tomographic plane of the region R_CC_jk.

In Fig. 13, a case is exemplified in which j = 4, k = 8, and the representative value P_R_T10_48 of the CC tomographic image T10 of the tomographic plane TF10 is the minimum value. In this case, the specifying unit 52 specifies the tomographic plane TF10 as the corresponding tomographic plane of the region R_CC_48 of the CC image. Then, as the information on the corresponding tomographic plane, i = 10 and the height 19 mm from the detection surface 22A are output to the storage controller 53. Although not illustrated, similarly for the MLO image, the specifying unit 52 specifies the corresponding tomographic plane of each region R_MLO_jk.

Here, as for the pixel value, a smaller value is assigned to the structure which has a higher absorption coefficient of the radiation 24 and appears whitish in the radiographic image. The selected location 70 is the lesion part such as calcification in many cases, and the lesion part has a relatively high absorption coefficient and has a relatively low pixel value. Accordingly, it can be said that the region R_Ti_jk in which the representative value P_R_Ti_jk is the minimum value is a region in which the probability that the lesion part, that is, the structure of the selected location 70 is present is relatively high. From the above consideration, it can be said that the method of specifying, as the corresponding tomographic plane, the tomographic plane TFi of the CC tomographic image Ti having the region R_Ti_jk in which the representative value P_R_Ti_jk is the minimum value is appropriate as a method of specifying the corresponding tomographic plane. In a case where a higher pixel value is assigned to the structure which has a higher absorption coefficient of the radiation 24, contrary to the above description, the tomographic plane TFi of the CC tomographic image Ti having the region R_Ti_jk in which the representative value P_R_TiJk is the maximum value is specified as the corresponding tomographic plane.

The location for specifying the corresponding tomographic plane in the specifying unit 52 may be one pixel instead of the above-described region. Further, after the corresponding tomographic plane is specified for each of the pixels, the corresponding tomographic plane of the region may be specified on the basis of the corresponding tomographic plane of each pixel. Specifically, the average value of the numerical values of i or the heights from the detection surface 22A of the corresponding tomographic planes of the pixels belonging to a certain region is obtained, and the tomographic plane indicated by the obtained average value is specified as the corresponding tomographic plane of the region.

The contour of the breast 23 shown in the image is extracted, and a location where the breast 23 is not shown is specified. Then, for the location where the breast 23 is not shown, the corresponding tomographic plane may not be specified. Alternatively, for the location where the breast 23 is not shown, the same tomographic plane such as the tomographic plane TF1 may be uniformly used as the corresponding tomographic plane.

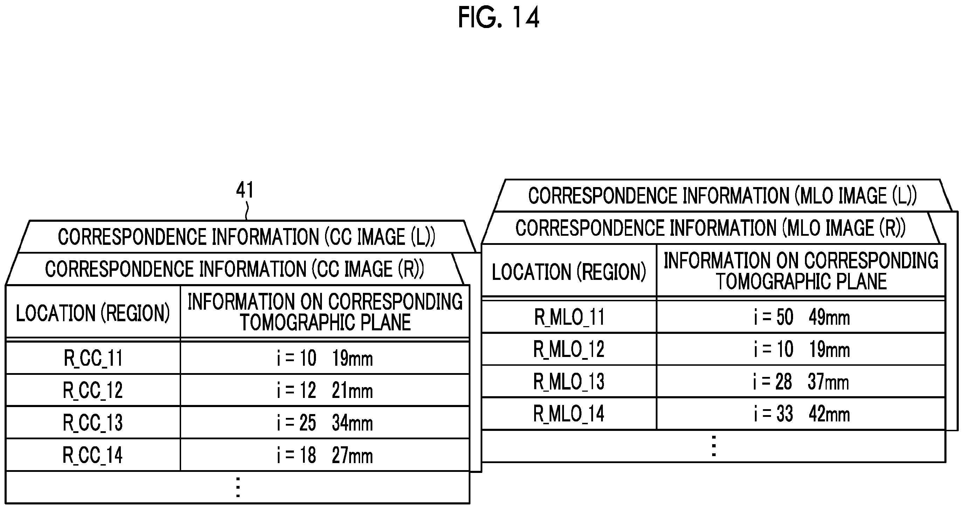

Since the specifying unit 52 specifies the corresponding tomographic plane for each of the CC image and the MLO image, a total of four kinds of information for the CC image (R), the CC image (L), the MLO image (R), and the MLO image (L) are stored as the correspondence information 41, as illustrated in Fig. 14. The correspondence information 41 is obtained such that information on the corresponding tomographic plane of the region is registered for each region.

As illustrated in Fig. 15, in the schema diagram template 42, one type of a schema diagram 60R for the right breast 23 and one type of a schema diagram 60L for the left breast 23 are stored. Since the schema diagrams 60R and 60L are bilaterally symmetrical and have the same basic configuration, in the following, only the schema diagram 60R will be described. In a case where it is not necessary to particularly distinguish the schema diagrams, the subscripts R and L are not used.

The schema diagram 60R has an outer circle 61R, an inner circle 62R, an X axis 63R, a Z axis 64R, an O axis 65R, and an ear-shaped portion 66R. The outer circle 61R represents the outer edge of the right breast 23, and the inner circle 62R represents the nipple of the right breast 23. The X axis 63R is an axis which passes through the centers of the outer circle 61R and the inner circle 62R and is parallel to the detection surface 22A in the CC imaging. The Z axis 64R is an axis which passes through the centers of the outer circle 61R and the inner circle 62R and is perpendicular to the detection surface 22A in the CC imaging. The O axis 65R is an axis which passes through the centers of the outer circle 61R and the inner circle 62R and is perpendicular to the detection surface 22A in the MLO imaging. As described above, since the imaging angle of the breast 23 in the MLO imaging is about 60°, the O axis 65R is inclined by 30° with respect to the X axis 63R.

The circular region formed by the outer circle 61R and the inner circle 62R is divided into four parts RA, RB, RC, and RD by the X axis 63R and the Z axis 64R. RA is the inner upper part, RB is the inner lower part, RC is the outer upper part, and RD is the outer lower part. Further, a part RE configured by the ear-shaped portion 66R is the axillary part.

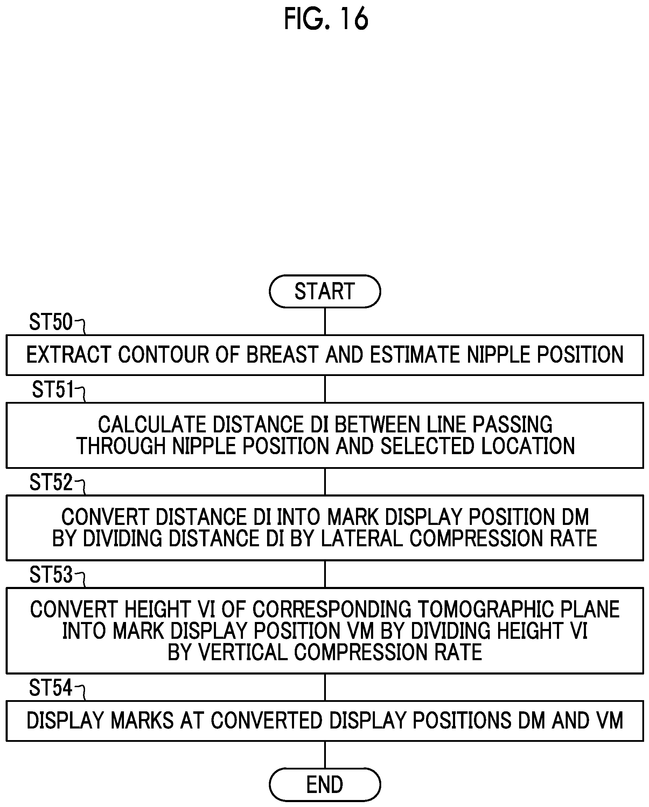

The generation unit 55 generates the schema diagram 60 with the mark 75 indicating the selected location 70 by the procedure illustrated in Fig. 16. That is, first, as illustrated in Step ST50, the contour of the breast 23 is extracted from one image of the CC image and the MLO image, which has accepted the selection instruction. Then, a nipple position NP (refer to Figs. 17A and 17B) is estimated on the basis of the extracted contour, by using a well-known image recognition technology.

Next, a distance DI (refer to Figs. 17A and 17B) between a line LV (refer to Figs. 17A and 17B) that passes through the nipple position NP and is perpendicular to a side of the chest wall and the selected location 70 is calculated (Step ST51). Subsequently, the calculated distance DI is divided by the lateral compression rate (refer to Fig. 18) of the breast 23 so that the distance DI is converted into a display position DM (refer to Figs. 19A and 19B) of the mark 75 on the schema diagram 60 (Step ST52). Similarly, a height VI from the detection surface 22A, which is included in the information on the corresponding tomographic plane from the acquisition unit 54, is divided by the vertical compression rate (refer to Fig. 18) of the breast 23 so that the height VI is converted into a display position VM (Figs. 19A and 19B) of the mark 75 on the schema diagram 60 (Step ST53). Finally, the mark 75 is displayed on the display positions DM and VM of the schema diagram 60, which are converted in Step ST 52 and Step ST53 (Step ST54).

Figs. 17A and 17B illustrate a state of calculating the distance DI with respect to the selected location 70. Fig. 17A illustrates a case of the CC image, and Fig. 17B illustrates a case of the MLO image. In Fig. 17A, a distance DI_CC between a line LV_CC, which passes through the nipple position NP and is perpendicular to a side of the chest wall, and the selected location 70 indicated by a cross is calculated. On the other hand, in Fig. 17B, a distance DI_MLO between a line LV_MLO, which passes through the nipple position NP and is perpendicular to a side of the chest wall, and the selected location 70 is calculated. The distance DI_CC has a positive value in a case where the selected location 70 is located below the line LV_CC as illustrated in Fig. 17A, that is, located in the inner upper part RA or the inner lower part RB, and the distance DI_CC has a negative value in a case where the selected location 70 is located above the line LV_CC, that is, located in the outer upper part RC or the outer lower part RD. Similarly, the distance DI_MLO has a positive value in a case where the selected location 70 is located above the line LV_MLO as illustrated in Fig. 17B, and the distance DI_MLO has a negative value in a case where the selected location 70 is located below the line LV_MLO.

Fig. 18 is a diagram for describing the lateral compression rate and the vertical compression rate. The lateral compression rate is represented by LB/LA in a case where the size of the breast 23 before the compression in the lateral direction (the direction of the X axis 63 or the direction perpendicular to the O axis 65) is set as LA and the size of the breast 23 after the compression in the lateral direction is set as LB. Similarly, the vertical compression rate is represented by VB/VA in a case where the size of the breast 23 before the compression in the vertical direction (the direction of the Z axis 64 or the direction perpendicular to the O axis 65) is set as VA and the size of the breast 23 after the compression in the vertical direction is set as VB.

In the embodiment, assuming that the lateral compression rate and the vertical compression rate are uniformly the same, fixed values are used for the rates. For example, the lateral compression rate = 1.5 and the vertical compression rate = 0.75. Accordingly, for example, in a case where the distance DI = 20 mm, the display position DM of the mark 75 is 20/1.5 ≈ 13.3 mm. Further, for example, in a case where the height VI = 28 mm, the display position VM of the mark 75 is 28/0.75 ≈ 37.3 mm.

In the embodiment, assuming that the imaging angle in the MLO imaging is uniformly the same, a fixed value is used. For example, the imaging angle = 60°.

Figs. 19A and 19B illustrate a state of displaying the mark 75 on the schema diagram 60R. Fig. 19A illustrates a case of the CC image, and Fig. 19B illustrates a case of the MLO image. First, in a case of the CC image in Fig. 19A, the mark 75 is displayed at a position which is separated from the Z axis 64R by DM_CC along the X axis 63R and is separated from the lowest point of the outer circle 61R by VM_CC along the Z axis 64R. In a case where DM_CC is a positive value, the mark 75 is disposed in the inner upper part RA or the inner lower part RB as illustrated in Fig. 19A. On the contrary, in a case where DM_CC is a negative value, the mark 75 is disposed in the outer upper part RC or the outer lower part RD.

On the other hand, in a case of the MLO image in Fig. 19B, the mark 75 is displayed at a position which is separated from the O axis 65R by DM_MLO along a tangent LW passing through the contact point between the outer circle 61R and the O axis 65R and is separated from the tangent LW by VM_MLO along the O axis 65R. In a case where DM_MLO is a positive value, the mark 75 is disposed above the O axis 65R as illustrated in Fig. 19B. On the contrary, in a case where DM_MLO is a negative value, the mark 75 is disposed below the O axis 65R.

Fig. 20 illustrates the transmission instruction acceptance screen 80 displayed on the display 34 by the display controller 56. On the transmission instruction acceptance screen 80, an image set display selection region 81 and a confirmation button 82 are provided. In the image set display selection region 81, the image sets 27 stored in the PACS server 11 are displayed in a list format so as to be alternatively selectable. In the image set display selection region 81, the imaging date and time, the common image ID, and the patient name of each image set 27 are displayed. The image set 27 selected in the image set display selection region 81 is displayed to be distinguishable from others as illustrated by hatching.

One of the image sets 27 displayed in the image set display selection region 81 is selected, and the confirmation button 82 is selected. In this manner, the transmission instruction of the selected image set 27 is given.

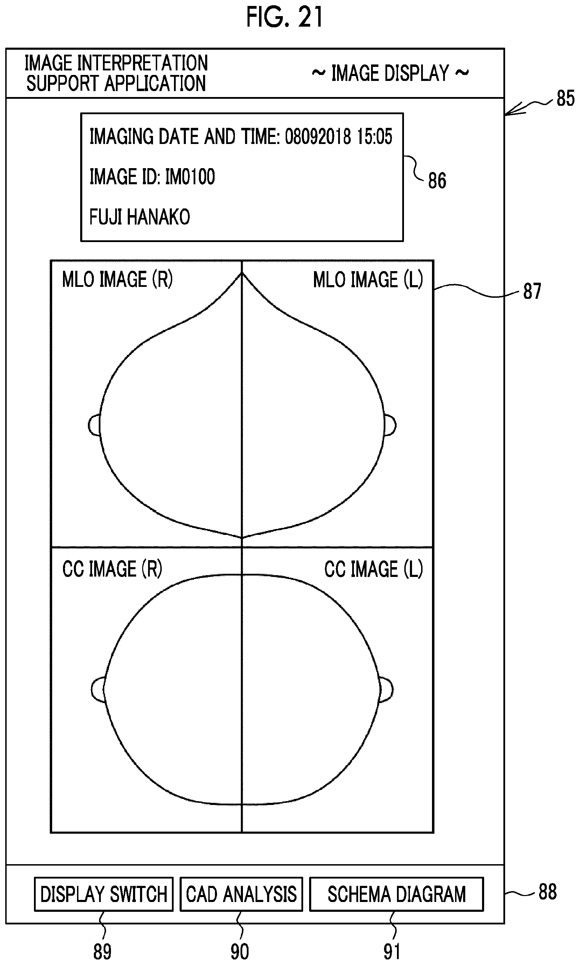

Fig. 21 illustrates an image display screen 85. The image display screen 85 is displayed on the display 34 by the display controller 56 instead of the transmission instruction acceptance screen 80 after the transmission instruction is given on the transmission instruction acceptance screen 80. On the image display screen 85, an accessory information display region 86, an image display region 87, and a button display region 88 are provided. In the accessory information display region 86, the imaging date and time, the common image ID, and the patient name are display. In the image display region 87, four images of the MLO image (R), the MLO image (L), the CC image (R), and the CC image (L) are displayed by being arranged vertically and laterally.

In the button display region 88, a display switch button 89, a computer-aided diagnosis (CAD) analysis button 90, and a schema diagram button 91 are arranged. In a case where the display switch button 89 is selected, the display of the image display region 87 is switched. For example, the illustrated parallel display of four images is switched to a single display of each of four images, a parallel display of the CC image (R) and the CC image (L), and a parallel display of the MLO image (R) and the MLO image (L). In a case where the CAD analysis button 90 is selected, various CAD analyses such as extraction of a lesion part and detection of the type of the structure are executed on the image displayed in the image display region 87. Then, an annotation or the like indicating the analysis result thereof is added to the image displayed in the image display region 87.

The schema diagram button 91 is a button for generating the schema diagram 60 with the mark 75. In a case where the schema diagram button 91 is selected, the display controller 56 displays the selection instruction acceptance screen 95 illustrated in Fig. 22 on the display 34.

In Fig. 22, in the selection instruction acceptance screen 95, an accessory information display region 96, an image display region 97, and a button display region 98 are provided, as in the image display screen 85 illustrated in Fig. 21. In the button display region 98, a display switch button 99, a cursor addition button 100, a cursor deletion button 101, and a confirmation button 102 are arranged.

In the image display region 97, first, the CC image (R) is displayed. The image to be displayed in the image display region 97 is switched, for example, from the CC image (R) to the CC image (L), the MLO image (R), and further the MLO image (L) by selecting the display switch button 99. In a case where the cursor addition button 100 is selected, one cursor 103 for performing the selection instruction is added to the image display region 97. On the contrary, in a case where the cursor deletion button 101 is selected, the added cursor 103 is deleted from the image display region 97. In this manner, the selection instruction can be performed a plurality of times on one image displayed in the image display region 97 by adding the cursor 103.

The cursor 103 can be moved to any location on the image displayed in the image display region 97. In a case where the cursor 103 is moved to a desired location by the user and the confirmation button 102 is selected, the selection instruction is given.

Fig. 23 illustrates the schema diagram display screen 110. The schema diagram display screen 110 is displayed on the display 34 by the display controller 56 instead of the selection instruction acceptance screen 95, after the selection instruction is given in the selection instruction acceptance screen 95. On the schema diagram display screen 110, an accessory information display region 111, a schema diagram display region 112, an image display region 113, and a button display region 114 are provided. In the schema diagram display region 112, the schema diagram 60 with the mark 75 generated by the generation unit 55 is displayed. In addition to the mark 75, a line LTF indicating the corresponding tomographic plane and an annotation 115 indicating the height of the corresponding tomographic plane are displayed on the schema diagram 60. In the image display region 113, in addition to the image on which the selection instruction is given, the tomographic image of the corresponding tomographic plane among the tomographic images obtained by performing the tomosynthesis imaging in the imaging method of the image on which the selection instruction is given is displayed. The cursor 103 indicating the selected location 70 is displayed in each image in the image display region 113. In Fig. 23, since the image on which the selection instruction is given is the CC image (R) and the height of the corresponding tomographic plane is 35 mm, the CC image (R) and the CC tomographic image (R) of which the height of the tomographic plane is 35 mm are displayed in the image display region 113. Further, in the tomographic image of the corresponding tomographic plane, a scale connecting the upper and lower limit values of the height from the detection surface 22A with a straight line and an arrow indicating the position on the scale of the height of the corresponding tomographic plane from the detection surface 22A may be displayed.

In the button display region 114, a schema diagram save button 116 and a return button 117 are arranged. In a case where the schema diagram save button 116 is selected, the schema diagram 60 displayed in the schema diagram display region 112 is saved in the corresponding image set 27 in the PACS server 11. In a case where the return button 117 is selected, the display returns from the schema diagram display screen 110 to the selection instruction acceptance screen 95.

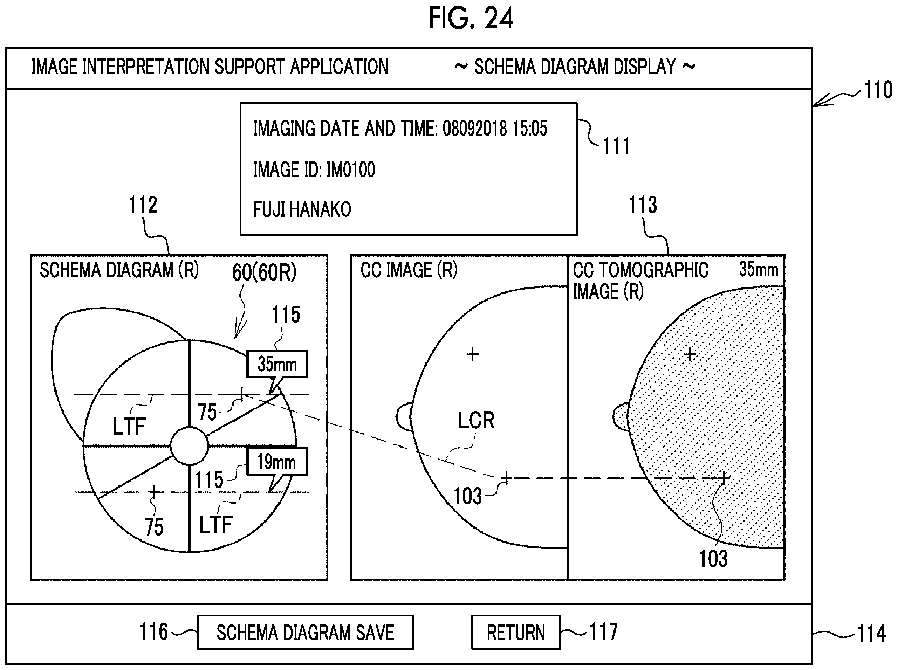

In a case where there are a plurality of selected locations 70 as illustrated in Fig. 24, the display controller 56 displays a line LCR indicating the correspondence relationship between the mark 75 and the cursor 103. The display controller 56 displays the line LCR in a case where any of the mark 75 of the schema diagram 60 or the cursor 103 of each image in the image display region 113 is selected. By doing so, which mark 75 corresponds to which cursor 103 (selected location 70) can be grasped at a glance.

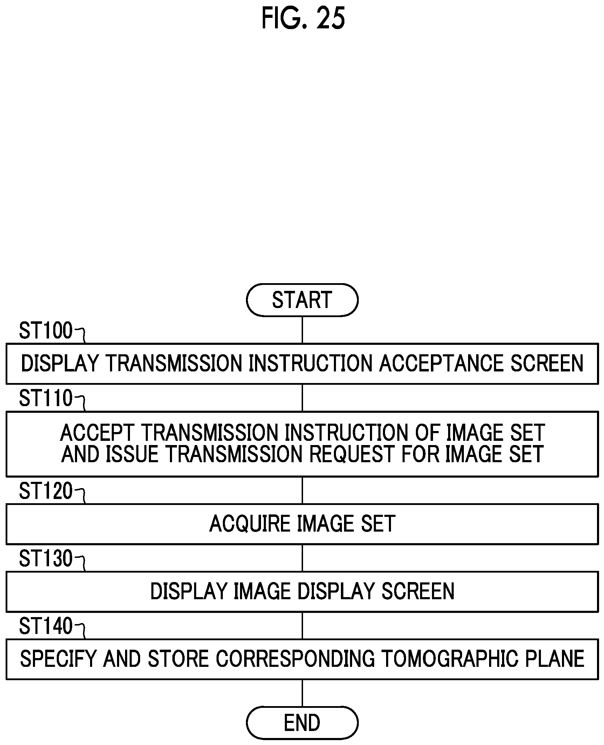

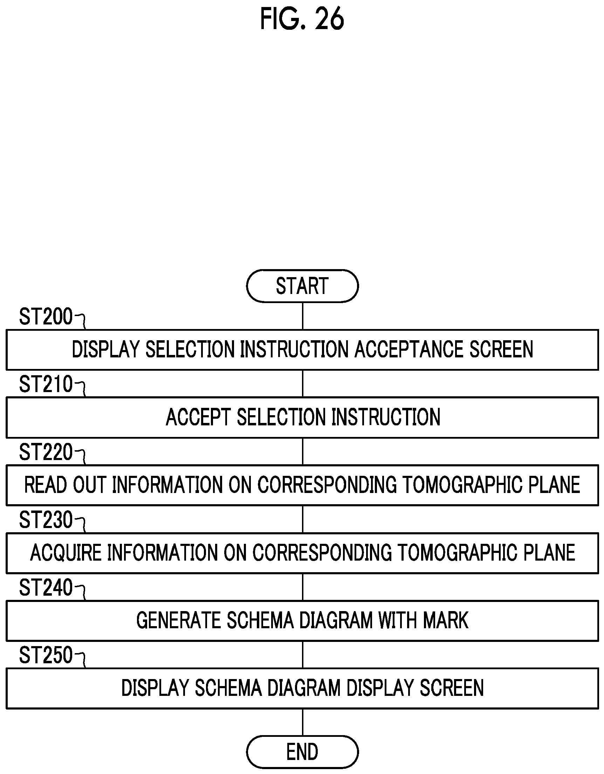

Next, the operation of the above configuration will be described with reference to the flowcharts illustrated in Figs. 25 and 26. First, the operation program 40 is activated, and thus the CPU 32 of the computer constituting the image interpretation support apparatus 12 functions as the processing units 50 to 56 as illustrated in Fig. 10. Then, as illustrated in Step ST100 of Fig. 25, the transmission instruction acceptance screen 80 illustrated in Fig. 20 is displayed on the display 34 by the display controller 56.

On the transmission instruction acceptance screen 80, in a case where one image set 27 in the image set display selection region 81 is selected and the confirmation button 82 is selected, the transmission instruction of the image set 27 is accepted by the acceptance unit 50. The transmission instruction is output from the acceptance unit 50 to the image acquisition unit 51. In this manner, the transmission request for the image set 27 is issued from the image acquisition unit 51 to the PACS server 11 (Step ST110).

Next, the image set 27 that is transmitted from the PACS server 11 in response to the transmission request is acquired by the image acquisition unit 51 (Step ST120). The image set 27 is output from the image acquisition unit 51 to the specifying unit 52 and the display controller 56.

The image display screen 85 illustrated in Fig. 21 is displayed on the display 34 by the display controller 56 on the basis of the image set 27 acquired by the image acquisition unit 51 (Step ST130). Further, the corresponding tomographic plane is specified by the specifying unit 52 as illustrated in Fig. 13. Then, as illustrated in Fig. 14, the information on the corresponding tomographic plane and the location corresponding thereto are stored as the correspondence information 41 in the storage device 30 by the storage controller 53 (Step ST140).

As illustrated in Step ST200 of Fig. 26, in a case where the schema diagram button 91 is selected on the image display screen 85, the selection instruction acceptance screen 95 illustrated in Fig. 22 is displayed on the display 34 by the display controller 56. On the selection instruction acceptance screen 95, in a case where the cursor 103 is moved to any location on the image displayed in the image display region 97 and the confirmation button 102 is selected, the selection instruction with the location of the cursor 103 as the selected location 70 is accepted by the acceptance unit 50 (Step ST210, acceptance step). The selection instruction is output from the acceptance unit 50 to the storage controller 53 and the generation unit 55.

The information on the corresponding tomographic plane corresponding to the selected location 70 selected by the selection instruction from the acceptance unit 50 is read from the correspondence information 41 by the storage controller 53 (Step ST220). The read information on the corresponding tomographic plane is output from the storage controller 53 to the acquisition unit 54. In this manner, the information on the corresponding tomographic plane is acquired by the acquisition unit 54 (Step ST230, acquisition step). The information on the corresponding tomographic plane is output from the acquisition unit 54 to the generation unit 55.

In this manner, before the selection instruction is accepted in the acceptance unit 50, the corresponding tomographic plane is specified by the specifying unit 52 in advance, and the information on the corresponding tomographic plane and the location thereof are stored as the correspondence information 41 in the storage device 30 in advance by the storage controller 53. Then, in a case where the selection instruction is accepted in the acceptance unit 50, the information on the corresponding tomographic plane corresponding to the selected location 70 is read from the correspondence information 41 and is output to the acquisition unit 54. Accordingly, in a case where the selection instruction is accepted in the acceptance unit 50, the information on the corresponding tomographic plane can be acquired by the acquisition unit 54 more quickly, and as a result, the display speed of the schema diagram display screen 110 is increased.

As illustrated in Figs. 16 to 19B, in the generation unit 55, the schema diagram 60 of the breast 23 with the mark 75 indicating the selected location 70 is generated on the basis of the positional information of the selected location 70 included in the selection instruction from the acceptance unit 50, the information on the corresponding tomographic plane from the acquisition unit 54, and the schema diagram template 42 of the storage device 30 (Step ST240, generation step). More specifically, the nipple position NP is estimated from the image, and the distance DI between the line LV, which passes through the nipple position NP and is perpendicular to a side of the chest wall, and the selected location 70 is calculated. Then, the distance DI and the height VI of the corresponding tomographic plane are converted into the display positions DM and VM of the mark 75 of the schema diagram 60 by being respectively divided by the lateral compression rate and the vertical compression rate. Finally, the mark 75 is displayed at the converted display positions DM and VM.

In this manner, in the generation unit 55, the display position of the mark 75 is decided on the basis of the distance DI between the line LV, which passes through the nipple position NP and is perpendicular to a side of the chest wall, and the selected location 70. Accordingly, the mark 75 can be displayed at a more accurate position.

The display position of the mark 75 may be decided on the basis of the distance between a line, which passes through, for example, the center of the image and is perpendicular to a side of the chest wall, and the selected location 70, instead of the line LV which passes through the nipple position NP and is perpendicular to a side of the chest wall. Although the accuracy of the display position of the mark 75 is inferior, the labor of the processing of estimating the nipple position NP by extracting the contour of the breast 23 can be saved.

The generated schema diagram 60 is output from the generation unit 55 to the display controller 56. Then, the schema diagram display screen 110 illustrated in Figs. 23 and 24 is displayed on the display 34 by the display controller 56 (Step ST250).

In this manner, the selection instruction for a location on one two-dimensional standard image (any one image of the CC image or the MLO image) is accepted in the acceptance unit 50, and the information on the corresponding tomographic plane corresponding to the selected location 70 for which the selection instruction is accepted in the acceptance unit 50 is acquired in the acquisition unit 54. Then, the schema diagram 60 with the mark 75 indicating the selected location 70 is generated in the generation unit 55 on the basis of the information on the corresponding tomographic plane acquired in the acquisition unit 54. Accordingly, the burden on the user can be reduced as compared with the technology in the related art in which the user has to make a selection instruction for the location on both the CC image and the MLO image.

The display controller 56 performs control to display, on the display 34, the schema diagram display screen 110 on which the schema diagram 60 with the mark 75 is displayed. Accordingly, the user can visually recognize the schema diagram 60 with the mark 75 via the schema diagram display screen 110. On the schema diagram display screen 110, the tomographic image of the corresponding tomographic plane among the tomographic images obtained by performing the tomosynthesis imaging in the same imaging method as that of the image on which the selection instruction is given is displayed. Accordingly, the schema diagram 60 with the mark 75 can be compared with the tomographic image of the corresponding tomographic plane among the tomographic images obtained by the tomosynthesis imaging in the same imaging method as the image on which the selection instruction is given, and the validity of the display position of the mark 75 can be checked.

### [Second Embodiment]

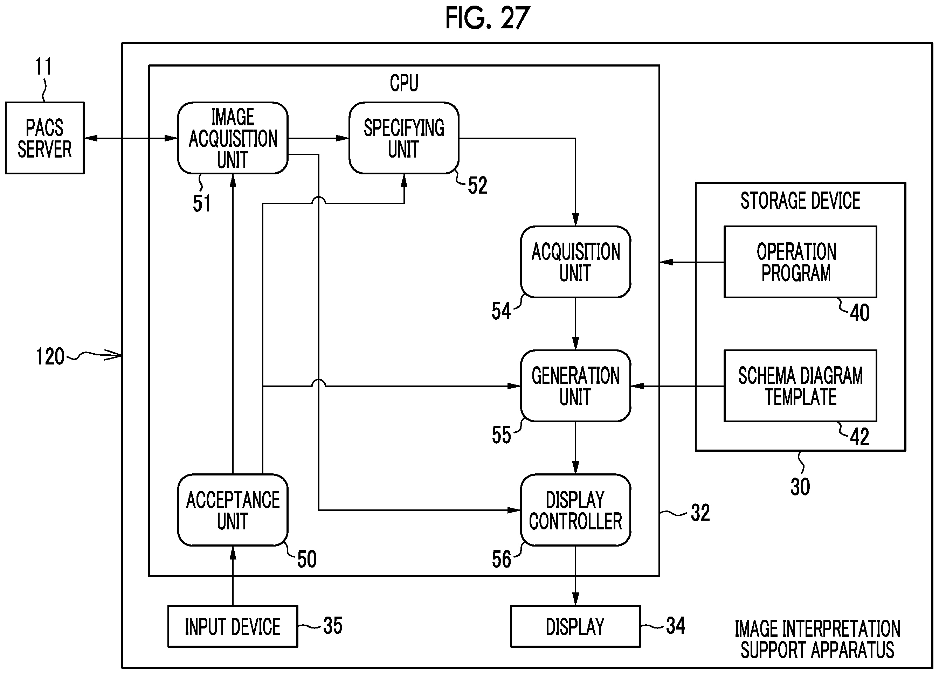

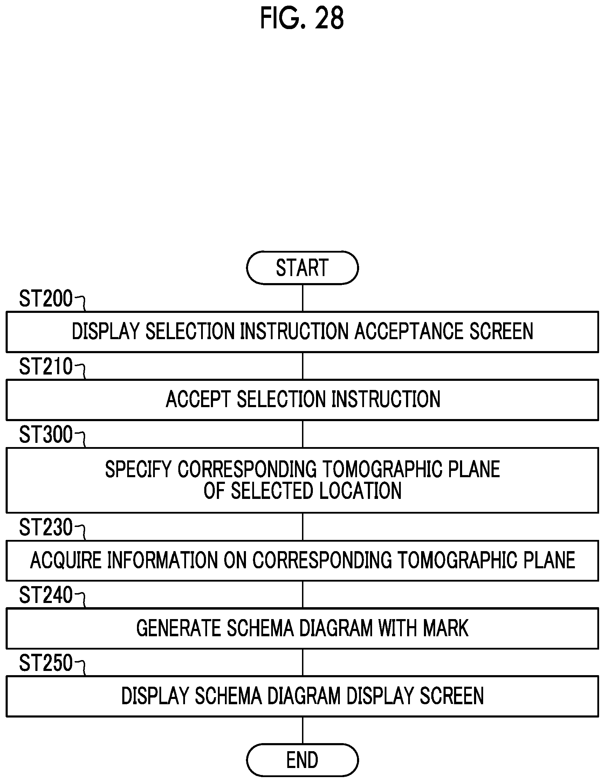

In this manner, in a second embodiment illustrated in Figs. 27 and 28, in a case where the selection instruction is accepted in the acceptance unit 50, the corresponding tomographic plane corresponding to the selected location 70 is specified.

In Fig. 27, in the storage device 30 of an image interpretation support apparatus 120 of the second embodiment, the operation program 40 and the schema diagram template 42 are stored, but the correspondence information 41 is not stored. In a case where the operation program 40 is activated, the CPU 32 functions as the processing units 50 to 52 and 54 to 56 except for the storage controller 53, among the processing units 50 to 56 illustrated in Fig. 10 of the first embodiment.

In this case, the acceptance unit 50 outputs the selection instruction to the specifying unit 52 and the generation unit 55. The specifying unit 52 is operated after the selection instruction is input from the acceptance unit 50. The specifying unit 52 specifies the corresponding tomographic plane corresponding to the selected location 70 included in the selection instruction from the acceptance unit 50, by using the method illustrated in Fig. 13, for example. The specifying unit 52 outputs the information on the specified corresponding tomographic plane to the acquisition unit 54. The acquisition unit 54 acquires the information on the corresponding tomographic plane.

Fig. 28 is a flowchart illustrating a processing procedure of the image interpretation support apparatus 120 of the second embodiment. The display of the selection instruction acceptance screen 95 in Step ST200 and the acceptance of the selection instruction by the acceptance unit 50 in Step ST210 are the same as those in the first embodiment.

The selection instruction is output from the acceptance unit 50 to the specifying unit 52 and the generation unit 55. In the specifying unit 52, the selection instruction from the acceptance unit 50 is received, and the corresponding tomographic plane corresponding to the selected location 70 is specified (Step ST300). The information on the specified corresponding tomographic plane is output to the acquisition unit 54. In this manner, the information on the corresponding tomographic plane is acquired by the acquisition unit 54 (Step ST230). Since the subsequent processing is the same as that of the first embodiment, the description thereof is omitted.

In this manner, in the second embodiment, in a case where the selection instruction is accepted in the acceptance unit 50, the specifying unit 52 specifies only the corresponding tomographic plane corresponding to the selected location 70. As in the first embodiment, in a case where the specifying unit 52 specifies the corresponding tomographic plane in advance for each location on the image before the selection instruction is accepted in the acceptance unit 50, the processing of specifying the corresponding tomographic plane of a location other than the selected location 70 is useless. However, in the second embodiment, it is possible to reduce such useless processing. Further, since it is not necessary to store the correspondence information 41 in the storage device 30, it is possible to reduce the capacity burden on the storage device 30.

### [Third Embodiment]

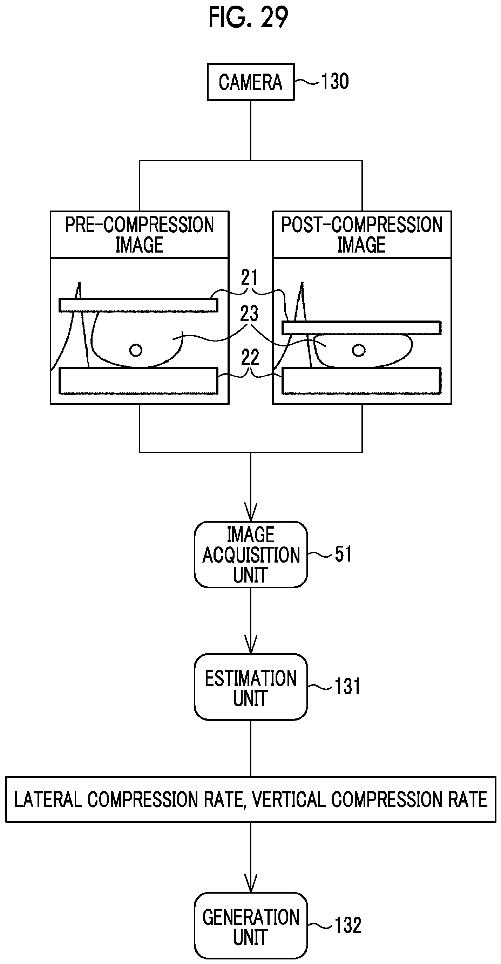

In a third embodiment illustrated in Fig. 29, the compression rate of the breast 23 is estimated, and the display position of the mark 75 is decided on the basis of the estimation result.

In Fig. 29, a mammography apparatus in the third embodiment comprises a camera 130 which captures frontal images of the breast 23 before and after the breast 23 is compressed by the compression plate 21 and the radiation detector 22 (hereinafter, referred to as a pre-compression image and a post-compression image). The camera 130 transmits the captured pre-compression image and post-compression image to the PACS server. The PACS server stores the pre-compression image and the post-compression image such that the images are included in the image set. The PACS server transmits the image set including the pre-compression image and the post-compression image to the image interpretation support apparatus in response to the transmission request. In the image interpretation support apparatus, the image set including the pre-compression image and the post-compression image is acquired in the image acquisition unit 51. The image acquisition unit 51 outputs the acquired image set to an estimation unit 131.

The estimation unit 131 estimates the compression rate of the breast 23 on the basis of the pre-compression image and the post-compression image. More specifically, the estimation unit 131 extracts the contour of the breast 23 shown on each of the pre-compression image and the post-compression image. Then, the estimation unit 131 obtains the size LA in the lateral direction and the size VA in the vertical direction illustrated in Fig. 18, from the contour of the breast 23 of the pre-compression image. Similarly, the estimation unit 131 obtains the size LB in the lateral direction and the size VB in the vertical direction illustrated in Fig. 18, from the contour of the breast 23 of the post-compression image. The estimation unit 131 calculates the lateral compression rate (= LB/LA) and the vertical compression rate (= VB/VA) of the breast 23 from the obtained sizes LA, LB, VA, and VB, and outputs the calculation result to a generation unit 132.

The generation unit 132 converts the distance DI into the display position DM of the mark 75 on the schema diagram 60 by dividing the distance DI by the lateral compression rate from the estimation unit 131. Further, the generation unit 132 converts the height VI into the display position VM of the mark 75 on the schema diagram 60 by dividing the height VI from the detection surface 22A, which is included in the information of the corresponding tomographic plane, by the vertical compression rate from the estimation unit 131.

In the first embodiment, fixed values are used on the assumption that the compression rates of the breast 23 are uniformly the same, but the compression rates of the breast 23 naturally vary depending on the patients. Accordingly, in a case where the compression rate of the breast 23 is estimated and the display position of the mark 75 is decided on the basis of the estimation result as in the third embodiment, the mark 75 can be displayed at a more accurate position in consideration of individual differences in the breast 23 of each patient.

Instead of obtaining the sizes LA, LB, VA, and VB from the contour of the breast 23 of the pre-compression image and the post-compression image, the user may measure and directly input the sizes LA, LB, VA, and VB.

### [Fourth Embodiment]

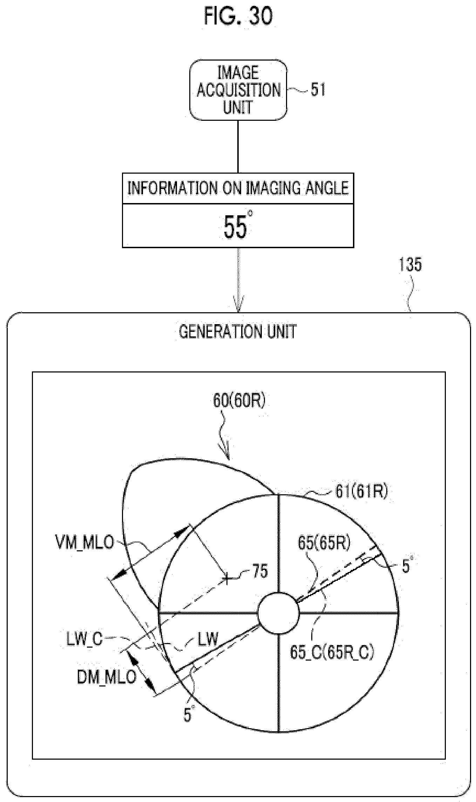

In a fourth embodiment illustrated in Fig. 30, the display position of the mark 75 is decided on the basis of the information on an imaging angle in the MLO imaging.

As illustrated in Fig. 8, the information on the imaging angle is included in the accessory information 28 of the MLO image. Therefore, as illustrated in Fig. 30, a generation unit 135 acquires the information on the imaging angle in a case where the image set 27 is acquired from the image acquisition unit 51. The generation unit 135 decides the display position of the mark 75 on the basis of the acquired information on the imaging angle.

More specifically, the generation unit 135 corrects the O axis 65 of the schema diagram 60 according to the imaging angle to obtain a corrected O axis 65_C. Further, the generation unit 135 also corrects the tangent LW passing through the contact point between the outer circle 61 and the O axis 65 in accordance with the correction of the O axis 65 to obtain a corrected tangent LW_C. Then, the display position of the mark 75 is decided on the basis of the corrected O axis 65_C and the corrected tangent LW_C.

Fig. 30 illustrates a case in which the imaging angle in the MLO imaging is 55°. In this case, the generation unit 135 corrects the O axis 65 to a position rotated by 5° counterclockwise, to obtain the corrected O axis 65_C. Further, the generation unit 135 sets a tangent passing through the contact point between the outer circle 61 and the corrected O axis 65_C as a corrected tangent LW_C. The generation unit 135 decides a position which is separated from the corrected O axis 65_C by DM_MLO along the corrected tangent LW_C and is separated from the corrected tangent LW_C by VM_MLO along the corrected O axis 65_C, as the display position of the mark 75.

The imaging angle varies depending on the size and shape of the breast 23, and is not always constant at 60°. However, as described above, the O axis 65 is provided according to the case where the imaging angle is 60°. Therefore, in a case where the imaging angle is other than 60°, in a case where the display position of the mark 75 is decided on the basis of the O axis 65 and the tangent LW as illustrated in Fig. 19B in the first embodiment, the display position deviates from the original position. Thus, in the fourth embodiment, the display position of the mark 75 is decided on the basis of the information on the imaging angle. In doing so, the mark 75 can be displayed at a more accurate position.

### [Fifth Embodiment]

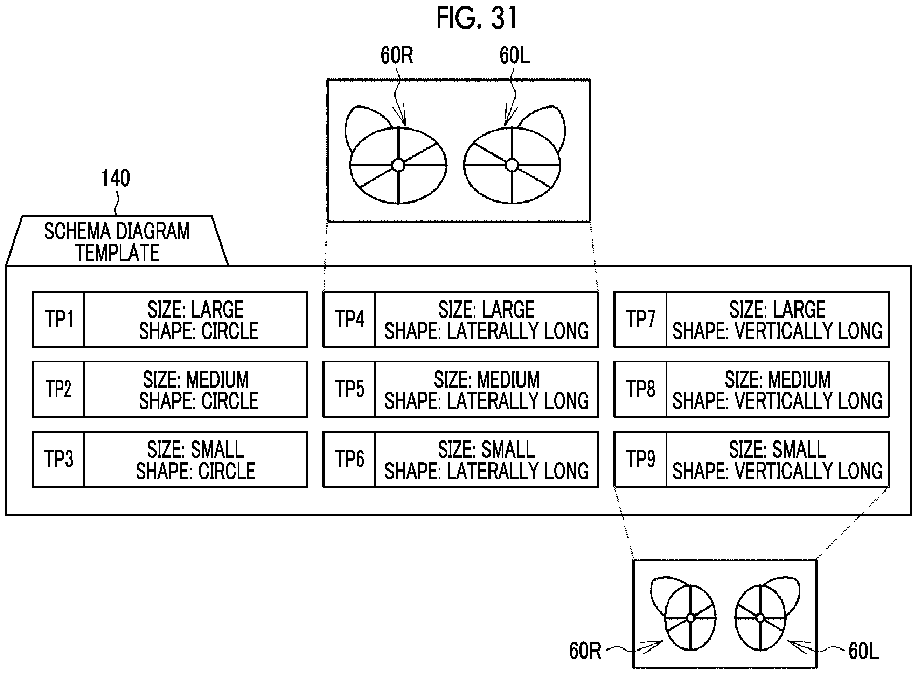

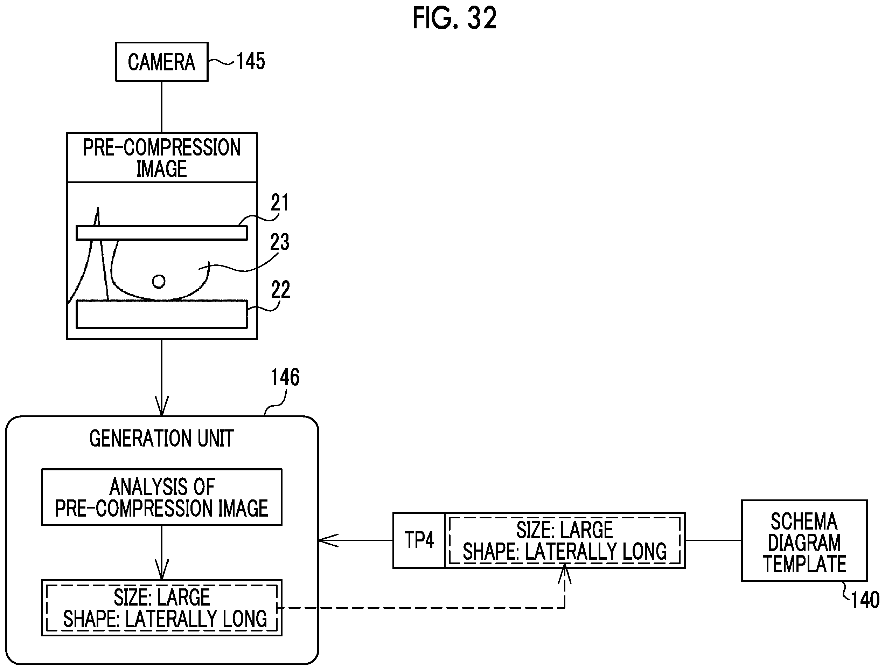

In the fifth embodiment illustrated in Figs. 31 and 32, the schema diagram 60 is changed in accordance with the size and shape of the breast 23.

As illustrated in Fig. 31, in a schema diagram template 140 in the fifth embodiment, a total of nine kinds of schema diagrams 60 of TP1 to TP9 are stored. TP1 to TP3 are for the breast 23 with a circular shape, TP4 to TP6 are for the breast 23 with a laterally long shape, and TP7 to TP9 are for the breast 23 with a vertically long shape. Further, TP1, TP4, and TP7 are for the breast 23 with a large size, TP2, TP5, and TP8 are for the breast 23 with a medium size, and TP3, TP6, and TP9 are for the breast 23 with a small size. As exemplified by TP9 or the like, each schema diagram 60 is composed of a set of the diagram for the right breast 23 and the diagram for the left breast 23.

In Fig. 32, similar to the third embodiment, a mammography apparatus in the fifth embodiment also comprises a camera 145 which captures a pre-compression image. Similar to the case in the third embodiment, the pre-compression image is included in the image set in the PACS server, is acquired by the image acquisition unit 51, and is output from the image acquisition unit 51 to a generation unit 146.

Similar to the third embodiment, the generation unit 146 extracts the contour of the breast 23 shown in the pre-compression image. Then, the generation unit 146 obtains the size LA in the lateral direction and the size VA in the vertical direction illustrated in Fig. 18, from the extracted contour. The generation unit 146 determines that the size is medium in a case where LA + VA is 15 cm to 20 cm, that the size is small in a case where LA + VA is smaller than 15 cm, and that the size is large in a case where LA + VA is larger than 20 cm. Further, for example, the generation unit 146 determines that the shape is circle in a case where LA/VA is 0.8 to 1.2, that the shape is vertically long in a case where LA/VA is smaller than 0.8, and that the shape is laterally long in a case where LA/VA is larger than 1.2. The generation unit 146 reads the schema diagram 60 that matches the determines size and shape, from the schema diagram template 140, and uses the schema diagram 60.

Fig. 32 illustrates a case in which the generation unit 146 determines that the size of the breast 23 is large and the shape of the breast 23 is laterally long. In this case, the schema diagram 60 of TP4 is read from the schema diagram template 140 and is used by the generation unit 146.

In this manner, in the fifth embodiment, the schema diagram 60 is changed in accordance with the size and shape of the breast 23, by the generation unit 146. Accordingly, the schema diagram 60 that more closely matches the size and shape of the breast 23 of the patient can be displayed.

Instead of determining the size and shape of the breast 23 on the basis of the pre-compression image captured by the camera 145, the user may directly input the size and shape of the breast 23. In this case, the user measures and inputs the size LA in the lateral direction and the size VA in the vertical direction. Alternatively, the user may input index values indicating the size and shape of the breast 23 such as cup and under-bust by asking the patient.

The schema diagram 60 may not be prepared as the template in advance. For example, the schema diagram 60 may be generated from scratch in accordance with the contour extracted from the pre-compression image. In doing so, the schema diagram 60 that more closely matches the size and shape of the breast 23 of the patient can be displayed, and it becomes easier to refer to the mark 75.

### [Sixth Embodiment]

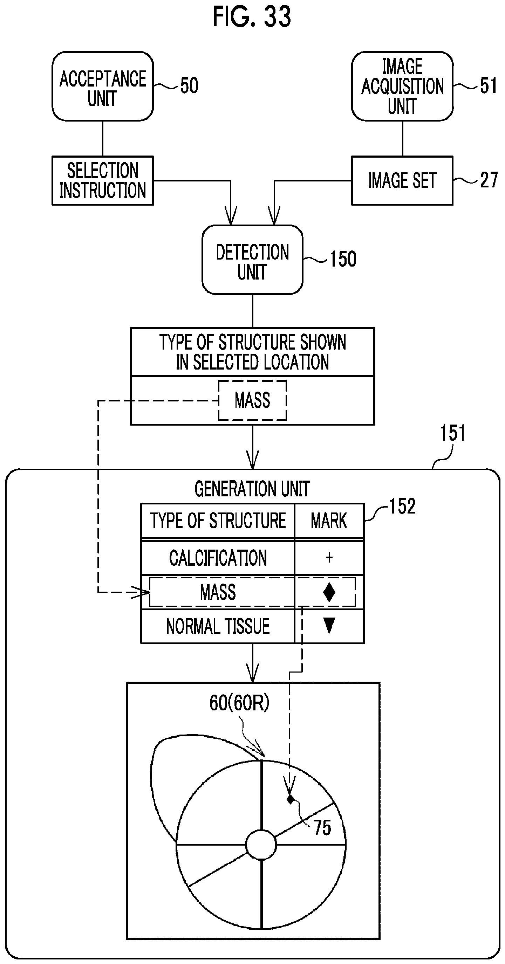

In a sixth embodiment illustrated in Fig. 33, a type of a structure shown in the selected location 70 is detected, and the mark 75 is changed in accordance with the detected type.

In Fig. 33, a detection unit 150 receives the selection instruction from the acceptance unit 50, and receives the image set 27 from the image acquisition unit 51. The detection unit 150 analyzes the selected location 70 of the image, on which the selection instruction is given, of the image set 27, using the CAD. Then, the detection unit 150 detects the type of the structure shown in the selected location 70. The detection unit 150 outputs the detected type of the structure to a generation unit 151.

The generation unit 151 changes the mark 75 in accordance with the type of the structure from the detection unit 150, according to a mark table 152. In the mark table 152, the type of the structure and the mark 75 corresponding thereto are registered. Specifically, a cross mark 75 is registered for a case where the type of the structure is calcification, a rhombus mark 75 is registered for a case where the type of the structure is a mass, and an inverted triangle mark 75 is registered for a case where the type of the structure is a normal tissue. A nodule and the like may be included in the type of the structure.

Fig. 33 illustrates a case where the detection unit 150 detects that the type of the structure shown in the selected location 70 is a mass. In this case, the generation unit 151 displays the rhombus mark 75 corresponding to the mass on the schema diagram 60.

In this manner, in the sixth embodiment, the type of the structure shown in the selected location 70 is detected by the detection unit 150, and the mark 75 is changed in accordance with the detected type by the generation unit 151. Accordingly, the user can immediately understand the type of the structure in the selected location 70 by simply looking at the mark 75.

### [Seventh Embodiment]

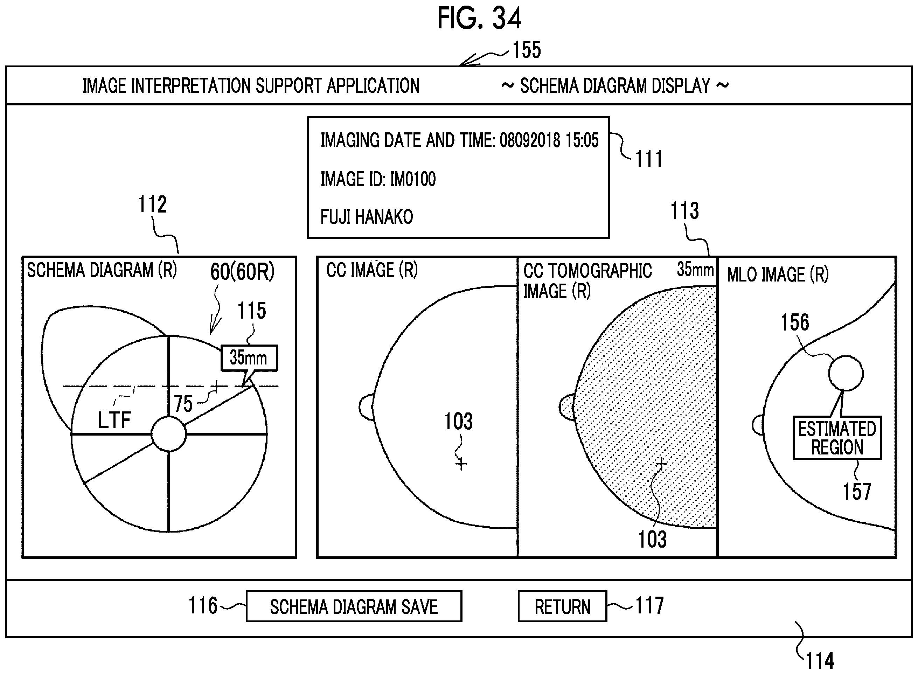

In a seventh embodiment illustrated in Fig. 34, an image obtained by imaging the breast 23 in an imaging method different from that of the two-dimensional standard image is also displayed.

A schema diagram display screen 155 illustrated in Fig. 34 has the same basic configuration as the schema diagram display screen 110 illustrated in Fig. 23. However, the schema diagram display screen 155 is different from the schema diagram display screen 110 in that, in addition to the CC image and the CC tomographic image, the MLO image is also displayed in the image display region 113.

In this case, the display controller 56 displays an estimated region 156 which is estimated to include the selected location 70, on the MLO image. Further, the display controller 56 displays an annotation 157 indicating that the region is the estimated region 156.

In the case of Fig. 34, the two-dimensional standard image is the CC image, and the image obtained by imaging the breast 23 in an imaging method different from that of the two-dimensional standard image is the MLO image. The display position of the estimated region 156 is not decided to be pinpointed as in the mark 75, and is a region estimated from the positional information of the selected location 70. Therefore, a certain margin is included in the estimated region 156, and the estimated region 156 is displayed as a circle larger than the mark 75 as illustrated in the drawing.

In this manner, in the seventh embodiment, the image obtained by imaging the breast 23 in an imaging method different from that of the two-dimensional standard image is also displayed on the display 34 by the display controller 56. Accordingly, the user can easily compare and interpret the respective images, and the image interpretation can be advanced efficiently.

Further, in the seventh embodiment, the estimated region 156 that is estimated to include the selected location 70 is displayed on the image obtained by imaging the breast 23 in an imaging method different from that of the two-dimensional standard image. Accordingly, even in the image obtained by imaging the breast 23 in an imaging method different from that of the two-dimensional standard image, the user can guess the selected location 70, and the image interpretation can be advanced more efficiently.

### [Eighth Embodiment]

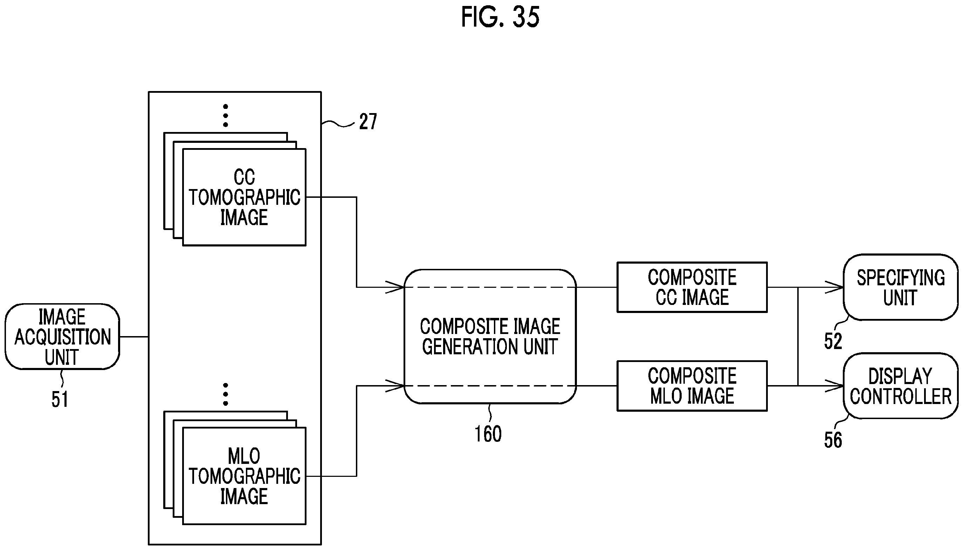

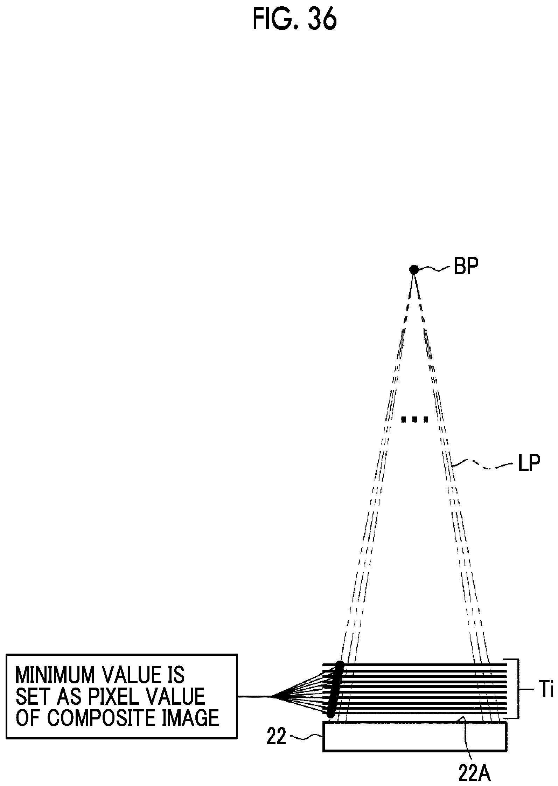

In an eighth embodiment illustrated in Figs. 35 and 36, a composite craniocaudal view image (composite CC image) is generated on the basis of the plurality of CC tomographic images obtained by the tomosynthesis imaging of the breast 23 in the CC imaging method. Further, a composite mediolateral oblique view image (composite MLO image) is generated on the basis of the plurality of MLO tomographic images obtained by the tomosynthesis imaging of the breast 23 in the MLO imaging method. Then, the composite CC image and the composite MLO image are used as the two-dimensional standard image on which the selection instruction is accepted.

In Fig. 35, a composite image generation unit 160 receives the image set 27 from the image acquisition unit 51. The composite image generation unit 160 generates a composite CC image on the basis of the plurality of CC tomographic images by using a well-known composite image generation technology such as a minimum intensity projection method. Similarly, the composite image generation unit 160 generates a composite MLO image on the basis of the plurality of MLO tomographic images by using a well-known composite image generation technology such as a minimum intensity projection method. The composite image generation unit 160 outputs the generated composite CC image and composite MLO image to the specifying unit 52 and the display controller 56.

As conceptually illustrated in Fig. 36, in the minimum intensity projection method, with a generation point of the radiation 24 at the position SP0 illustrated in Fig. 4 as a reference point BP, a projection line LP is drawn from the reference point BP to each location of the detection surface 22A of the radiation detector 22. Then, the minimum value among the pixel values of the locations of the tomographic images Ti through which the projection line LP passes is used as a pixel value of the composite image. In a case where a higher pixel value is assigned to the structure which has a higher absorption coefficient of the radiation 24, a maximum intensity projection method is adopted instead of the minimum intensity projection method.

The specifying unit 52 specifies the corresponding tomographic plane of each location of the composite CC image and the composite MLO image, instead of the CC image and the MLO image. Further, the display controller 56 displays the composite CC image and the composite MLO image on the image display screen 85 and the selection instruction acceptance screen 95, instead of the CC image and the MLO image. That is, the composite CC image and the composite MLO image are used as the two-dimensional standard image on which the selection instruction is accepted.

In this manner, in the eighth embodiment, the composite CC image generated on the basis of the plurality of CC tomographic images obtained by the tomosynthesis imaging of the breast 23 in the CC imaging method is used as the two-dimensional standard image. Further, the composite MLO image generated on the basis of the plurality of MLO tomographic images obtained by imaging the breast 23 by the tomosynthesis imaging in the MLO imaging method is used as the two-dimensional standard image. Accordingly, the CC imaging in Step ST10 illustrated in Fig. 6 and the MLO imaging in Step ST20 illustrated in Fig. 7 in the first embodiment are unnecessary, and it is possible to reduce the amount of radiation exposure of the patient. Further, the imaging time can be shortened.

In a case where the composite MLO image is used as the two-dimensional standard image, similar to the fourth embodiment, the display position of the mark 75 may be decided on the basis of the information on the imaging angle in the tomosynthesis imaging in the MLO imaging method.

In Figs. 23, 24, and 34, an example of displaying the schema diagram 60 and the like for the right breast 23 is illustrated, but it goes without saying that the schema diagram 60 and the like for the left breast 23 may be displayed. Further, the schema diagram 60 and the like for the right breast 23 and the schema diagram 60 and the like for the left breast 23 may be displayed side by side.

Instead of or in addition to displaying the schema diagram 60 with the mark 75, the schema diagram 60 with the mark 75 may be printed or may be attached to an e-mail and sent.

The schema diagram may be used as three-dimensional computer graphics. In this case, the generation unit 55 also obtains the distance between a side of the chest wall and the selected location 70 in addition to the distance DI between the line LV, which passes through the nipple position NP and is perpendicular to the side of the chest wall and the selected location 70. Then, the display position of the mark 75 in a depth direction (direction of the line LV) of the schema diagram of the three-dimensional computer graphics is decided on the basis of the obtained distance.

The hardware configuration of the computer constituting the image interpretation support apparatus can be variously modified. For example, in order to improve the processing capacity and reliability, the image interpretation support apparatus may be constituted by a plurality of computers that are separated from each other as hardware. Specifically, the functions of the acceptance unit 50, the image acquisition unit 51, the specifying unit 52, and the storage controller 53 and the functions of the acquisition unit 54, the generation unit 55, and the display controller 56 may be distributed in two computers. In this case, the two computers constitute the image interpretation support apparatus.

The operation program 40 is installed in mammography apparatus 10 or the PACS server 11. Then, all or a part of the processing units constructed in the CPU 32 of the image interpretation support apparatus in the embodiments described above may be constructed in the mammography apparatus 10 or the PACS server 11, and the mammography apparatus 10 or the PACS server 11 may be operated as the image interpretation support apparatus.

In this manner, the hardware configuration of the computer can be appropriately changed according to the required performance, such as processing capacity, safety, or reliability. Further, in order to secure the safety and the reliability, without being limited to hardware, an application program such as the operation program 40 may be duplicated or may be distributed and stored in a plurality of storage devices.

In the embodiments described above, the PACS server 11 is used in one medical facility, but the PACS server 11 may be used in a plurality of medical facilities. In this case, the PACS server 11 is communicably connected to a plurality of mammography apparatuses 10 and image interpretation support apparatuses installed in a plurality of medical facilities via a wide area network (WAN) such as the Internet or a public communication network. Then, the image from the mammography apparatus 10 in each medical facility and the transmission request from the image interpretation support apparatus in each medical facility are transmitted to the PACS server 11 via the WAN, the image from the mammography apparatus 10 in each medical facility is managed, and the image set 27 is transmitted to the image interpretation support apparatus in each medical facility. In this case, the installation location and management entity of the PACS server 11 may be a data center managed by a company that is different from the medical facility, or may be one of the plurality of medical facilities.

In the embodiments described above, for example, the following various processors can be used as the hardware structure of processing units executing various processes such as the acceptance unit 50, the image acquisition unit 51, the specifying unit 52, the storage controller 53, the acquisition unit 54, the generation units 55, 132, 135, 146, and 151, the display controller 56, the estimation unit 131, the detection unit 150, and the composite image generation unit 160. The various processors include, for example, a programmable logic device (PLD) that is a processor of which the circuit configuration can be changed after manufacture, such as a field-programmable gate array (FPGA), and a dedicated electric circuit that is a processor having a dedicated circuit configuration designed to execute a specific process, such as an application specific integrated circuit (ASIC), in addition to the CPU 32 that is a general-purpose processor which executes software (operation program 40) to function as various processing units as described above.

One processing unit may be configured by one of the various processors or a combination of the same or different kinds of two or more processors (for example, a combination of a plurality of FPGAs or a combination of a CPU and an FPGA). In addition, a plurality of processing units may be configured by one processor.

As an example where a plurality of processing units are configured by one processor, first, there is a form where one processor is configured by a combination of one or more CPUs and software as typified by a computer, such as a client and a server, and this processor functions as a plurality of processing units. Second, there is a form where a processor fulfilling the functions of the entire system including a plurality of processing units by one integrated circuit (IC) chip as typified by a system on chip (SoC) or the like is used. In this manner, various processing units are configured by using one or more of the above-described various processors as hardware structures.

In addition, specifically, an electric circuit (circuitry) obtained by combining circuit elements, such as semiconductor elements, can be used as the hardware structure of the various processors.

From the above description, the invention described in Additional remark 1 described below can be grasped.

### [Additional remark 1]

An image interpretation support apparatus comprising:
an acceptance processor that accepts a selection instruction of a location on one two-dimensional standard image having information on a breast;
an acquisition processor that acquires information on a corresponding tomographic plane corresponding to a selected location, which is the location on which the selection instruction is accepted by the acceptance processor, among a plurality of tomographic planes of the breast, the corresponding tomographic plane being specified on the basis of a plurality of tomographic images in the plurality of tomographic planes obtained by tomosynthesis imaging of the breast; and
a generation processor that generates a schema diagram of the breast with a mark indicating the selected location on the basis of the information on the corresponding tomographic plane acquired by the acquisition processor.

In the technology of the present disclosure, it is also possible to appropriately combine the above-described various embodiments and various modification examples. Further, without being limited to the embodiments described above, various configurations can be adopted without departing from the scope. Further, in addition to the program, the technology of the present disclosure also extends to a storage medium that stores the program non-temporarily.

The contents described and illustrated above are detailed descriptions of a part relating to the technology of the present disclosure, and are merely examples of the technology of the present disclosure. For example, the above description regarding the configuration, function, action, and effect is a description regarding an example of the configuration, function, action, and effect of a part of the technology of the present disclosure. Accordingly, it goes without saying that with respect to the contents described and illustrated above, unnecessary parts may be deleted, new elements may be added or replaced within a range not departing from the scope of the technology of the present disclosure. In addition, in order to avoid complications and facilitate understanding of a part relating to the technology of the present disclosure, in the contents described and illustrated above, descriptions regarding common technical knowledge and the like that do not require any explanation to enable the implementation of the technology of the present disclosure are omitted.

## Claims

1. An image interpretation support apparatus (12) comprising:
a reception unit (50) that receives a selection instruction of a location on one two-dimensional standard image having information on a breast;
an acquisition unit (54) that acquires information on a corresponding tomographic plane corresponding to a selected location, which is the location of the selection instruction received by the reception unit, among a plurality of tomographic planes of the breast, the corresponding tomographic plane being specified on the basis of a plurality of tomographic images in the plurality of tomographic planes obtained by tomosynthesis imaging of the breast;
a detection unit (150) that detects a type of a structure shown in the selected location;
a generation unit (55, 151) that generates a schema diagram of the breast with a mark indicating the selected location on the basis of the information on the corresponding tomographic plane acquired by the acquisition unit,
wherein the generation unit (55, 151) changes the mark in the schema diagram in accordance with the type detected by the detection unit (150) and according to the correspondence of the mark to the type of the structure, and
wherein the two-dimensional standard image is a simple image obtained by performing simple imaging of the breast, or a composite image generated based on the plurality of tomographic images.

2. The image interpretation support apparatus (12) according to claim 1, further comprising:
a specifying unit (52) that specifies the corresponding tomographic plane on the basis of the plurality of tomographic images.

3. The image interpretation support apparatus (12) according to claim 2,
wherein the specifying unit (52) specifies the corresponding tomographic plane for each location of the two-dimensional standard image before receiving the selection instruction at the reception unit, and
the image interpretation support apparatus further comprising
a storage controller (53) that performs control to store, as correspondence information (41), the information on the corresponding tomographic plane specified in the specifying unit (52) and a location of the corresponding tomographic plane specified by the specifying unit (52), in a storage unit (30), and that outputs the information on the corresponding tomographic plane corresponding to the selected location to the acquisition unit (54) by reading the information on the corresponding tomographic plane from the correspondence information (41).

4. The image interpretation support apparatus (12) according to claim 2,
wherein the specifying unit (52) specifies the corresponding tomographic plane corresponding to the selected location in a case in which the reception unit (50) receives the selection instruction.

5. The image interpretation support apparatus (12) according to any one of claims 1 to 4,
wherein the generation unit (55) determines a display position of the mark on the basis of a distance between a line, which passes through a nipple position shown in the two-dimensional standard image and is perpendicular to a side of a chest wall, and the selected location.

6. The image interpretation support apparatus (12) according to any one of claims 1 to 5, further comprising:
an estimation unit (131) that estimates a compression rate of the breast,
wherein the generation unit (132) determines a display position of the mark on the basis of an estimation result of the estimation unit (132).

7. The image interpretation support apparatus (12) according to any one of claims 1 to 6,
wherein the two-dimensional standard image is at least one of
a craniocaudal view image obtained by imaging the breast in a craniocaudal direction,
a mediolateral oblique view image obtained by imaging the breast in a mediolateral oblique direction,
a composite craniocaudal view image generated on the basis of a plurality of craniocaudal view tomographic images obtained by the tomosynthesis imaging of the breast in a craniocaudal view imaging method, or
a composite mediolateral oblique view image generated on the basis of a plurality of mediolateral oblique view tomographic images obtained by the tomosynthesis imaging of the breast in a mediolateral oblique view imaging method.

8. The image interpretation support apparatus (12) according to claim 7,
wherein the generation unit (135) determines a display position of the mark on the basis of information on an imaging angle in the mediolateral oblique view imaging in a case in which the two-dimensional standard image is the mediolateral oblique view image or the composite mediolateral oblique view image.

9. The image interpretation support apparatus (12) according to any one of claims 1 to 8,
wherein the generation unit (146) changes the schema diagram in accordance with a size and a shape of the breast.

10. The image interpretation support apparatus (12) according to any one of claims 1 to 9, further comprising:
a display controller (56) that performs control to display the schema diagram on a display unit.

11. The image interpretation support apparatus according to claim 10,
wherein the display controller (56) performs control to display, in addition to the schema diagram, on the display unit, the tomographic image of the corresponding tomographic plane among the tomographic images obtained by the tomosynthesis imaging of the breast in the same imaging method as an imaging method of the two-dimensional standard image.

12. The image interpretation support apparatus according to claim 11,
wherein the display controller (56) performs control to further display, on the display unit, an image obtained by imaging the breast in a different imaging method from the imaging method of the two-dimensional standard image.

13. The image interpretation support apparatus according to claim 12,
wherein the display controller (56) displays an estimated region that is estimated to include the selected location, on the image obtained by imaging the breast in the different imaging method from the imaging method of the two-dimensional standard image.

14. A non-transitory storage medium storing an operation program (40) that causes a computer to perform operation processing of an image interpretation support apparatus (12), the operation processing comprising:
receiving (ST210) a selection instruction of a location on one two-dimensional standard image having information on a breast;
acquiring (ST230) information on a corresponding tomographic plane corresponding to a selected location, which is the location of the received selection instruction, among a plurality of tomographic planes of the breast, the corresponding tomographic plane being specified on the basis of a plurality of tomographic images in the plurality of tomographic planes obtained by tomosynthesis imaging of the breast;
detecting a type of a structure shown in the selected location;
generating (ST240) a schema diagram of the breast with a mark indicating the selected location on the basis of the information on the acquired corresponding tomographic plane; and
changing the mark in the schema diagram in accordance with the detected type of the structure and according to the correspondence of the mark to the type of the structure,
wherein the two-dimensional standard image is a simple image obtained by performing simple imaging of the breast, or a composite image generated based on the plurality of tomographic images.

15. An operation method of an image interpretation support apparatus (12), the operation method comprising:
receiving (ST210) a selection instruction of a location on one two-dimensional standard image having information on a breast;
acquiring (ST230) information on a corresponding tomographic plane corresponding to a selected location, which is the location of the received selection instruction, among a plurality of tomographic planes of the breast, the corresponding tomographic plane being specified on the basis of a plurality of tomographic images in the plurality of tomographic planes obtained by tomosynthesis imaging of the breast;
detecting a type of a structure shown in the selected location;
generating (ST240) a schema diagram of the breast with a mark indicating the selected location on the basis of the information on the acquired corresponding tomographic plane;
changing the mark in the schema diagram in accordance with the detected type of the structure and according to the correspondence of the mark to the type of the structure,
wherein the two-dimensional standard image is a simple image obtained by performing simple imaging of the breast, or a composite image generated based on the plurality of tomographic images.

## Patentansprüche

1. Bildinterpretationsunterstützungsvorrichtung (12), umfassend:
eine Empfangseinheit (50), die eine Auswahlanweisung für eine Stelle auf einem zweidimensionalen Standardbild mit Informationen über eine Brust empfängt;
eine Erfassungseinheit (54), die Informationen über eine entsprechende tomographische Ebene erfasst, die einem ausgewählten Ort entspricht, der der Ort der von der Empfangseinheit empfangenen Auswahlanweisung ist, aus einer Vielzahl von tomographischen Ebenen der Brust, wobei die entsprechende tomographische Ebene auf der Grundlage einer Vielzahl von tomographischen Bildern in der Vielzahl von tomographischen Ebenen spezifiziert wird, die durch Tomosynthese-Bildgebung der Brust erhalten werden;
eine Erkennungseinheit (150), die einen Typ einer Struktur erkennt, die an dem ausgewählten Ort angezeigt wird;
eine Erzeugungseinheit (55, 151), die ein Schemadiagramm der Brust mit einer Markierung erzeugt, die die ausgewählte Stelle auf der Grundlage der von der Erfassungseinheit erfassten Informationen über die entsprechende tomographische Ebene anzeigt,
wobei die Erzeugungseinheit (55, 151) die Markierung in dem Schemadiagramm entsprechend dem von der Erfassungseinheit (150) erfassten Typ und entsprechend der Übereinstimmung der Markierung mit dem Typ der Struktur ändert, und
wobei das zweidimensionale Standardbild ein einfaches Bild ist, das durch die Durchführung einer einfachen Bildgebung der Brust erhalten wird, oder ein zusammengesetztes Bild, das auf der Grundlage der mehreren tomographischen Bilder erzeugt wird.

2. Die Bildinterpretationsunterstützungsvorrichtung (12) nach Anspruch 1, die ferner Folgendes umfasst:
eine Spezifizierungseinheit (52), die die entsprechende tomographische Ebene auf der Grundlage der Vielzahl von tomographischen Bildern spezifiziert.

3. Bildinterpretationsunterstützungsvorrichtung (12) nach Anspruch 2,
wobei die Spezifizierungseinheit (52) die entsprechende tomographische Ebene für jede Stelle des zweidimensionalen Standardbildes spezifiziert, bevor sie den Auswahlbefehl an der Empfangseinheit empfängt, und
wobei die Bildinterpretationsunterstützungsvorrichtung Bildinterpretation ferner umfasst
eine Speichersteuerung (53), die eine Steuerung durchführt, um die Information über die entsprechende tomographische Ebene, die in der Spezifizierungseinheit (52) spezifiziert ist, und einen Ort der entsprechenden tomographischen Ebene, der durch die Spezifizierungseinheit (52) spezifiziert ist, als Korrespondenzinformation (41) in einer Speichereinheit (30) zu speichern, und die die Information über die entsprechende tomographische Ebene, die dem ausgewählten Ort entspricht, an die Erfassungseinheit (54) ausgibt, indem sie die Information über die entsprechende tomographische Ebene aus der Korrespondenzinformation (41) liest.

4. Bildinterpretationsunterstützungsvorrichtung (12) nach Anspruch 2,
wobei die Spezifizierungseinheit (52) die entsprechende tomographische Ebene spezifiziert, die dem ausgewählten Ort in einem Fall entspricht, in dem die Empfangseinheit (50) die Auswahlanweisung empfängt.

5. Bildinterpretationsunterstützungsvorrichtung (12) nach einem der Ansprüche 1 bis 4,
wobei die Erzeugungseinheit (55) eine Anzeigeposition der Markierung auf der Grundlage eines Abstands zwischen einer Linie, die durch eine in dem zweidimensionalen Standardbild dargestellte Brustwarzenposition verläuft und senkrecht zu einer Seite einer Brustwand ist, und der ausgewählten Stelle bestimmt.

6. Bildinterpretationsunterstützungsvorrichtung (12) nach einem der Ansprüche 1 bis 5, ferner umfassend:
eine Schätzungseinheit (131), die eine Kompressionsrate der Brust schätzt,
wobei die Erzeugungseinheit (132) eine Anzeigeposition der Marke auf der Grundlage eines Schätzergebnisses der Schätzeinheit (132) bestimmt.

7. Bildinterpretationsunterstützungsvorrichtung (12) nach einem der Ansprüche 1 bis 6,
wobei das zweidimensionale Standardbild mindestens eines der folgenden ist
ein Bild aus kraniokaudaler Sicht, das durch Aufnahme der Brust in kraniokaudaler Richtung gewonnen wird,
ein Bild in mediolateraler Schrägansicht, das durch Aufnahme der Brust in mediolateraler Schrägrichtung gewonnen wird,
ein zusammengesetztes Bild aus kraniokaudaler Sicht, das auf der Grundlage einer Vielzahl von tomografischen Bildern aus kraniokaudaler Sicht erzeugt wird, die durch die Tomosynthese-Bildgebung der Brust in einem Bildgebungsverfahren aus kraniokaudaler Sicht erhalten wurden, oder
ein zusammengesetztes Bild der mediolateralen Schrägansicht, das auf der Grundlage einer Vielzahl von tomographischen Bildern der mediolateralen Schrägansicht erzeugt wird, die durch die Tomosynthese-Bildgebung der Brust in einem Bildgebungsverfahren der mediolateralen Schrägansicht erhalten wurden.

8. Bildinterpretationsunterstützungsvorrichtung (12) nach Anspruch 7,
wobei die Erzeugungseinheit (135) eine Anzeigeposition der Markierung auf der Grundlage von Informationen über einen Abbildungswinkel in der mediolateralen Schrägsichtabbildung in einem Fall bestimmt, in dem das zweidimensionale Standardbild das mediolaterale Schrägsichtbild oder das zusammengesetzte mediolaterale Schrägsichtbild ist.

9. Bildinterpretationsunterstützungsvorrichtung (12) nach einem der Ansprüche 1 bis 8,
wobei die Erzeugungseinheit (146) das Schemadiagramm in Abhängigkeit von der Größe und Form der Brust ändert.

10. Bildinterpretationsunterstützungsvorrichtung (12) nach einem der Ansprüche 1 bis 9, ferner umfassend:
eine Anzeigesteuerung (56), die eine Steuerung zur Anzeige des Schemadiagramms auf einer Anzeigeeinheit durchführt.

11. Bildinterpretationsunterstützungsvorrichtung nach Anspruch 10,
wobei die Anzeigesteuerung (56) eine Steuerung durchführt, um zusätzlich zu dem Schemadiagramm auf der Anzeigeeinheit das tomographische Bild der entsprechenden tomographischen Ebene unter den tomographischen Bildern anzuzeigen, die durch die Tomosynthese-Bildgebung der Brust in demselben Bildgebungsverfahren wie ein Bildgebungsverfahren des zweidimensionalen Standardbildes erhalten werden.

12. Bildinterpretationsunterstützungsvorrichtung nach Anspruch 11,
wobei die Anzeigesteuerung (56) eine Steuerung durchführt, um auf der Anzeigeeinheit weiterhin ein Bild anzuzeigen, das durch Abbilden der Brust in einem anderen Abbildungsverfahren als dem Abbildungsverfahren des zweidimensionalen Standardbildes erhalten wurde.

13. Bildinterpretationsunterstützungsvorrichtung nach Anspruch 12,
wobei die Anzeigesteuerung (56) einen geschätzten Bereich, von dem angenommen wird, dass er die ausgewählte Stelle einschließt, auf dem Bild anzeigt, das durch Abbilden der Brust mit einem anderen Abbildungsverfahren als dem Abbildungsverfahren des zweidimensionalen Standardbildes erhalten wurde.

14. Ein nicht-transitorisches Speichermedium, das ein Betriebsprogramm (40) speichert, das einen Computer veranlasst, eine Betriebsverarbeitung einer Bildinterpretationsunterstützungsvorrichtung (12) durchzuführen, wobei die Betriebsverarbeitung umfasst:
Empfangen (ST210) einer Auswahlanweisung für eine Stelle auf einem zweidimensionalen Standardbild mit Informationen über eine Brust;
Erfassen (ST230) von Informationen über eine entsprechende tomographische Ebene, die einem ausgewählten Ort, der der Ort der empfangenen Auswahlanweisung ist, unter einer Vielzahl von tomographischen Ebenen der Brust entspricht, wobei die entsprechende tomographische Ebene auf der Grundlage einer Vielzahl von tomographischen Bildern in der Vielzahl von tomographischen Ebenen spezifiziert wird, die durch Tomosynthese-Bildgebung der Brust erhalten werden;
Erkennung des Typs einer Struktur, die an dem ausgewählten Ort angezeigt wird;
Erzeugen (ST240) eines Schemadiagramms der Brust mit einer Markierung, die die ausgewählte Stelle auf der Grundlage der Informationen über die erfasste entsprechende tomographische Ebene anzeigt; und
Änderung der Markierung im Schemadiagramm entsprechend dem erkannten Typ der Struktur und entsprechend der Übereinstimmung der Markierung mit dem Typ der Struktur,
wobei das zweidimensionale Standardbild ein einfaches Bild ist, das durch die Durchführung einer einfachen Bildgebung der Brust erhalten wird, oder ein zusammengesetztes Bild, das auf der Grundlage der mehreren tomographischen Bilder erzeugt wird.

15. Betriebsverfahren für eine Bildinterpretationsunterstützungsvorrichtung (12), wobei das Betriebsverfahren umfasst:
Empfangen (ST210) einer Auswahlanweisung für eine Stelle auf einem zweidimensionalen Standardbild mit Informationen über eine Brust;
Erfassen (ST230) von Informationen über eine entsprechende tomographische Ebene, die einem ausgewählten Ort, der der Ort der empfangenen Auswahlanweisung ist, unter einer Vielzahl von tomographischen Ebenen der Brust entspricht, wobei die entsprechende tomographische Ebene auf der Grundlage einer Vielzahl von tomographischen Bildern in der Vielzahl von tomographischen Ebenen spezifiziert wird, die durch Tomosynthese-Bildgebung der Brust erhalten werden;
Erkennung des Typs einer Struktur, die an dem ausgewählten Ort angezeigt wird;
Erzeugen (ST240) eines Schemadiagramms der Brust mit einer Markierung, die die ausgewählte Stelle auf der Grundlage der Informationen über die erfasste entsprechende tomographische Ebene anzeigt;
Änderung der Markierung im Schemadiagramm entsprechend dem erkannten Typ der Struktur und entsprechend der Übereinstimmung der Markierung mit dem Typ der Struktur,
wobei das zweidimensionale Standardbild ein einfaches Bild ist, das durch die Durchführung einer einfachen Bildgebung der Brust erhalten wird, oder ein zusammengesetztes Bild, das auf der Grundlage der mehreren tomographischen Bilder erzeugt wird.

## Revendications

1. Appareil de support d'interprétation d'images (12) comprenant :
une unité de réception (50) qui reçoit une instruction de sélection d'un emplacement sur une image standard bidimensionnelle contenant des informations sur un sein ;
une unité d'acquisition (54) qui acquiert des informations sur un plan tomographique correspondant à un emplacement sélectionné, qui est l'emplacement de l'instruction de sélection reçue par l'unité de réception, parmi une pluralité de plans tomographiques du sein, le plan tomographique correspondant étant spécifié sur la base d'une pluralité d'images tomographiques dans la pluralité de plans tomographiques obtenus par imagerie de tomosynthèse du sein ;
une unité de détection (150) qui détecte le type d'une structure représentée à l'endroit sélectionné ;
une unité de génération (55, 151) qui génère un schéma du sein avec une marque indiquant l'emplacement sélectionné sur la base des informations sur le plan tomographique correspondant acquises par l'unité d'acquisition,
l'unité de génération (55, 151) modifie la marque dans le schéma en fonction du type détecté par l'unité de détection (150) et en fonction de la correspondance de la marque avec le type de la structure, et
l'image standard bidimensionnelle est une image simple obtenue en réalisant une imagerie simple du sein, ou une image composite générée à partir de la pluralité d'images tomographiques.

2. Appareil de support d'interprétation d'images (12) selon la revendication 1, comprenant en outre :
une unité de spécification (52) qui spécifie le plan tomographique correspondant sur la base de la pluralité d'images tomographiques.

3. Appareil de support d'interprétation d'images (12) selon la revendication 2, dans lequel
l'unité de spécification (52) spécifie le plan tomographique correspondant pour chaque emplacement de l'image standard bidimensionnelle avant de recevoir l'instruction de sélection de l'unité de réception, et
l'appareil d'aide à l'interprétation des images comprend en outre
un contrôleur de stockage (53) qui effectue une commande pour stocker, en tant qu'informations de correspondance (41), les informations sur le plan tomographique correspondant spécifiées dans l'unité de spécification (52) et un emplacement du plan tomographique correspondant spécifié par l'unité de spécification (52), dans une unité de stockage (30), et qui émet les informations sur le plan tomographique correspondant à l'emplacement sélectionné vers l'unité d'acquisition (54) en lisant les informations sur le plan tomographique correspondant à partir des informations de correspondance (41).

4. Appareil de support d'interprétation d'images (12) selon la revendication 2, dans lequel
l'unité de spécification (52) spécifie le plan tomographique correspondant à l'emplacement sélectionné dans le cas où l'unité de réception (50) reçoit l'instruction de sélection.

5. Appareil de support d'interprétation d'images (12) selon l'une quelconque des revendications 1 à 4, dans lequel
l'unité de génération (55) détermine une position d'affichage de la marque sur la base d'une distance entre une ligne, qui passe par une position de mamelon montrée dans l'image standard bidimensionnelle et qui est perpendiculaire à un côté de la paroi thoracique, et l'emplacement sélectionné.

6. Appareil de support d'interprétation d'images (12) selon l'une quelconque des revendications 1 à 5, comprenant en outre :
une unité d'estimation (131) qui estime le taux de compression du sein,
l'unité de génération (132) détermine une position d'affichage de la marque sur la base d'un résultat d'estimation de l'unité d'estimation (132).

7. Appareil de support d'interprétation d'images (12) selon l'une quelconque des revendications 1 à 6,
dans lequel l'image standard bidimensionnelle est au moins l'une des suivantes
une vue crânio-caudale obtenue par imagerie du sein dans le sens crânio-caudal,
une vue oblique médiane obtenue par l'imagerie du sein dans une direction oblique médiane,
une image composite de la vue craniocaudale générée sur la base de plusieurs images tomographiques de la vue craniocaudale obtenues par l'imagerie de tomosynthèse du sein dans une méthode d'imagerie de la vue craniocaudale, ou
une image composite de la vue oblique médiane générée sur la base d'une pluralité d'images tomographiques de la vue oblique médiane obtenues par l'imagerie de tomosynthèse du sein dans le cadre d'une méthode d'imagerie de la vue oblique médiane.

8. Appareil de support d'interprétation d'images (12) selon la revendication 7, dans lequel
l'unité de génération (135) détermine une position d'affichage de la marque sur la base d'informations relatives à un angle d'imagerie dans la vue oblique médiane dans le cas où l'image standard bidimensionnelle est l'image oblique médiane ou l'image oblique médiane composite.

9. Appareil de support d'interprétation d'images (12) selon l'une quelconque des revendications 1 à 8, dans lequel
l'unité de génération (146) modifie le schéma en fonction de la taille et de la forme du sein.

10. Appareil de support d'interprétation d'images (12) selon l'une quelconque des revendications 1 à 9, comprenant en outre :
un contrôleur d'affichage (56) qui commande l'affichage du schéma sur une unité d'affichage.

11. Appareil de support d'interprétation d'images selon la revendication 10, dans lequel
le contrôleur d'affichage (56) commande l'affichage, en plus du schéma, sur l'unité d'affichage, de l'image tomographique du plan tomographique correspondant parmi les images tomographiques obtenues par tomosynthèse du sein selon la même méthode d'imagerie qu'une méthode d'imagerie de l'image standard bidimensionnelle.

12. Appareil de support d'interprétation d'images selon la revendication 11, dans lequel
le contrôleur d'affichage (56) commande l'affichage, sur l'unité d'affichage, d'une image obtenue par imagerie du sein selon une méthode d'imagerie différente de la méthode d'imagerie de l'image standard bidimensionnelle.

13. Appareil de support d'interprétation d'images selon la revendication 12, dans lequel
le contrôleur d'affichage (56) affiche une région estimée comme comprenant l'emplacement sélectionné, sur l'image obtenue par l'imagerie du sein selon la méthode d'imagerie différente de la méthode d'imagerie de l'image standard bidimensionnelle.

14. Support de stockage non transitoire stockant un programme d'opération (40) qui amène un ordinateur à effectuer un traitement d'opération d'un appareil de support d'interprétation d'image (12), le traitement d'opération comprenant :
recevoir (ST210) une instruction de sélection d'un emplacement sur une image standard bidimensionnelle contenant des informations sur un sein ;
acquérir (ST230) des informations sur un plan tomographique correspondant à un emplacement sélectionné, qui est l'emplacement de l'instruction de sélection reçue, parmi une pluralité de plans tomographiques du sein, le plan tomographique correspondant étant spécifié sur la base d'une pluralité d'images tomographiques dans la pluralité de plans tomographiques obtenus par imagerie de tomosynthèse du sein ;
la détection d'un type de structure représenté à l'endroit sélectionné ;
générer (ST240) un schéma du sein avec une marque indiquant l'emplacement sélectionné sur la base des informations du plan tomographique correspondant acquis ; et
modification de la marque dans le schéma en fonction du type de structure détecté et en fonction de la correspondance entre la marque et le type de structure,
l'image standard bidimensionnelle est une image simple obtenue en réalisant une imagerie simple du sein, ou une image composite générée à partir de la pluralité d'images tomographiques.

15. Méthode de fonctionnement d'un appareil de support d'interprétation d'images (12), la méthode de fonctionnement comprenant :
recevoir (ST210) une instruction de sélection d'un emplacement sur une image standard bidimensionnelle contenant des informations sur un sein ;
acquérir (ST230) des informations sur un plan tomographique correspondant à un emplacement sélectionné, qui est l'emplacement de l'instruction de sélection reçue, parmi une pluralité de plans tomographiques du sein, le plan tomographique correspondant étant spécifié sur la base d'une pluralité d'images tomographiques dans la pluralité de plans tomographiques obtenus par imagerie de tomosynthèse du sein ;
la détection d'un type de structure représenté à l'endroit sélectionné ;
générer (ST240) un schéma du sein avec une marque indiquant l'emplacement sélectionné sur la base des informations du plan tomographique correspondant acquis ;
modification de la marque dans le schéma en fonction du type de structure détecté et en fonction de la correspondance entre la marque et le type de structure,
l'image standard bidimensionnelle age est une image simple obtenue par imagerie simple du sein, ou une image composite générée à partir de la pluralité d'images tomographiques.
